(19) Europäisches Patentamt
European Patent Office
Office européen des brevets

(11) Veröffentlichungsnummer: **0 043 940**
**B1**

(12) # EUROPÄISCHE PATENTSCHRIFT

(45) Veröffentlichungstag der Patentschrift: **12.09.84**

(51) Int. Cl.³: **C 07 D 265/22**, A 61 K 31/535

(21) Anmeldenummer: **81104787.7**

(22) Anmeldetag: **22.06.81**

(54) **3,1-Benzoxazin-2-one, ihre Herstellung und Verwendung.**

(30) Priorität: **12.07.80 DE 3026534**

(43) Veröffentlichungstag der Anmeldung: **20.01.82 Patentblatt 82/3**

(45) Bekanntmachung des Hinweises auf die Patenterteilung: **12.09.84 Patentblatt 84/37**

(84) Benannte Vertragsstaaten: **AT BE CH DE FR IT LI LU NL SE**

(56) Entgegenhaltungen:
**EP - A - 0 002 867**
**EP - A - 0 005 848**
**EP - A - 0 008 653**
**DE - A - 2 609 645**

**Die Akte enthält technische Angaben, die nach dem Eingang der Anmeldung eingereicht wurden und die nicht in dieser Patentschrift enthalten sind.**

(73) Patentinhaber: **C.H. BOEHRINGER SOHN, Postfach 200, *D-6507 Ingelheim am Rhein (DE)***

(72) Erfinder: **Mentrup, Anton, Dr., Steinern Strasse 25, D-6503 Mainz-Kastel (DE)**
Erfinder: **Schromm, Kurt, Dr., In der Dörrwies 35, D-6507 Ingelheim (DE)**
Erfinder: **Renth, Ernst-Otto, Dr., Frankenstrasse 11, D-6507 Ingelheim (DE)**
Erfinder: **Hoefke, Wolfgang, Dr., Rosselstrasse 21, D-6200 Wiesbaden (DE)**
Erfinder: **Gaida, Wolfram, Dr., Paul-Clemen-Strasse 14, D-6507 Ingelheim (DE)**
Erfinder: **Streller, Ilse, Dr., Waldstrasse 16, D-6534 Stromberg (DE)**
Erfinder: **Fügner, Armin, Dr., Im Hippel 31, D-6535 Gau-Algesheim (DE)**

**EP 0 043 940 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 3,1-Benzoxazin-2-one der allgemeinen Formel

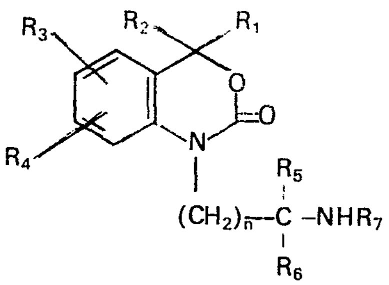

(I),

ihre Herstellung nach an sich bekannten Verfahren und ihre Verwendung als Wirkstoffe von Arzneimitteln bzw. als Zwischenprodukte für die Herstellung solcher Wirkstoffe.

In der Formel I und im folgenden bedeuten

$R_1$ und $R_2$, die gleich oder verschieden sein können, Wasserstoff oder $(C_1-C_4)$-Alkyl

$R_3$ und $R_4$, die gleich oder verschieden sein können, Wasserstoff, Fluor, Chlor, Hydroxy, Methyl, Ethyl, $(C_1-C_4)$-Alkoxy oder auch gemeinsam Methylendioxy,

$R_5$ und $R_6$, die gleich oder verschieden sein können, Wasserstoff oder Methyl,

$R_7$ die Gruppe

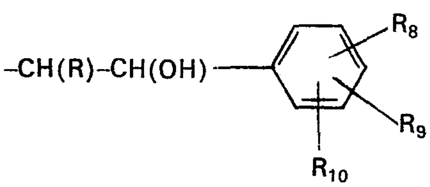

(II),

R Wasserstoff oder $(C_1-C_4)$-Alkyl,

$R_8$ Fluor, Chlor, $(C_1-C_4)$-Alkyl, $(C_1-C_4)$-Alkylthio, Hydroxymethyl oder eine der Gruppen $CONHR_{11}$, $SO_2NHR_{11}$, $OR_{12}$, Methoxycarbonyl, Ethoxycarbonyl oder $NHSO_2CH_3$,

$R_9$ Wasserstoff, Fluor, Chlor oder $OR_{12}$,

$R_{10}$ Wasserstoff, Amino, Methyl, Methoxy oder Chlor,

$R_{11}$ Wasserstoff, Methyl, Ethyl oder Hydroxyethyl

$R_{12}$ Wasserstoff, $(C_1-C_4)$-Alkyl, $(C_1-C_4)$-Alkyl-CO, $C_6H_5-CH_2$ oder $C_6H_5-CO$ und

n 1, 2 oder 3.

Soweit die genannten Substituenten Alkylreste darstellen oder enthalten, können diese gegebenenfalls geradkettig oder verzweigt sein, d.h. sie stehen für Methyl, Ethyl, n-Propyl, Isopropyl, n-Butyl, Isobutyl, sec.-Butyl, tert.-Butyl. «Aryl» bedeutet einen Phenylrest.

Hervorzuheben sind die folgenden Substituentenbedeutungen (im Rahmen der obigen Definitionen):

$R_1/R_2$: $CH_3/CH_3$ oder $CH_3/H$,
$R_3$: H, OH, $OCH_3$,
$R_4$: H,
$R_5/R_6$: $CH_3/CH_3$, $CH_3/H$ oder H/H,
$R_7$: obige Bedeutung,
R: H, $CH_3$, $C_2H_5$,
$R_8$: $CONHR_{11}$, $SO_2NHR_{11}$, $NHSO_2CH_3$, $OR_{12}$, F, Cl, COOAlkyl, $CH_2OH$,
$R_9$: $OR_{12}$, F, Cl, H,
$R_{10}$: H, $NH_2$, $CH_3$, $OCH_3$, Cl,
$R_{11}$: H, $CH_3$, $C_2H_5$,
$R_{12}$: H, Aryl-$CH_2$, Alkyl-CO,
n: 1 oder 2

Die erfindungsgemässen Verbindungen können als Racemate, in Form diastereomeren Antipodenpaare sowie in Form der einzelnen Enantiomeren vorliegen, jeweils als freie Basen oder als Säureadditionssalze.

Aus der deutschen Patentanmeldung 26 09 645 sind Phenylethanolamine bekannt, deren Aminogruppe u.a. durch einen heterocyclisch substituierten Alkylrest der Formel

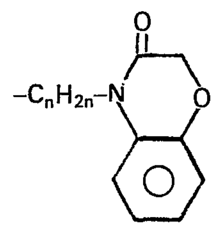

worin n eine ganze Zahl zwischen 2 und 6 bedeutet, substituiert sein kann. Die Verbindungen wirken gefässerweiternd und können auch als Broncholytika und Antihypertonika angewendet werden. Sie sind jedoch den erfindungsgemässen Verbindungen hinsichtlich ihres Wirkungsprofils unterlegen, weil sie als Broncholytika deutlich weniger selektiv wirken und als Blutdrucksenker einen störenden Einfluss auf die Herzfrequenz zeigen.

Die europäische Patentanmeldung 0 008 653 beschreibt ebenfalls Phenylethanolamine, die am Stickstoff einen heterocyclisch substituierten Alkylrest enthalten. Den erfindungsgemässen Verbindungen am nächsten stehen diejenigen Verbindungen, die den Rest der Formel

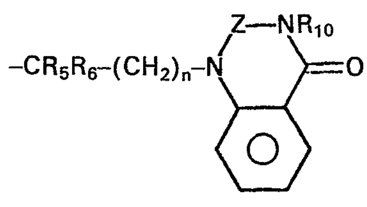

enthalten.

Hierin steht n für eine ganze Zahl von 1 bis 4, $R_5$ und $R_6$ sind Wasserstoff oder Methyl, $R_{10}$ ist u.a. Wasserstoff oder Alkyl, und Z ist $CH_2$ oder CO. Diese Verbindungen haben insbesondere broncholytische, spasmolytische und antiallergische Wirkung. Auch den Verbindungen nach dieser europäischen Anmeldung sind die erfindungsgemässen Verbindungen deutlich überlegen, insbesondere bezüglich der Selektivität bei der Broncholyse, aber auch im Verhältnis Blutdrucksenkung zu Änderung der Herzfrequenz.

Die Herstellung der neuen Verbindungen der Formel I kann nach an sich bekannten Verfahren erfolgen:

1) Reduktion einer Verbindung der allgemeinen Formel

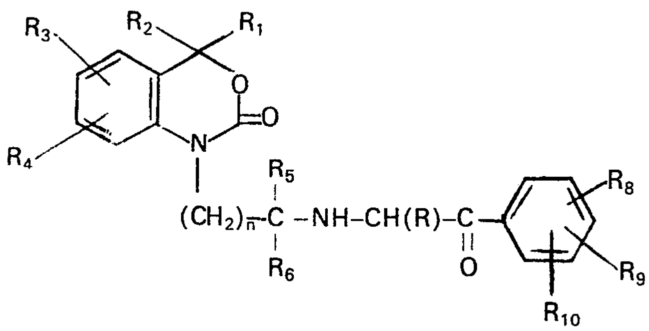

(II)

in der R, $R_1$–$R_{10}$ und n die obige Bedeutung haben.

Als Reduktionsmittel dienen vorzugsweise komplexe Hydride, insbesondere Natriumborhydrid oder Wasserstoff/Hydrierungskatalysatoren. Als solche Katalysatoren kommen vor allem Platin, Palladium und Nickel in Betracht.

2) Reduktive Alkylierung von Aminoverbindungen der allgemeinen Formel

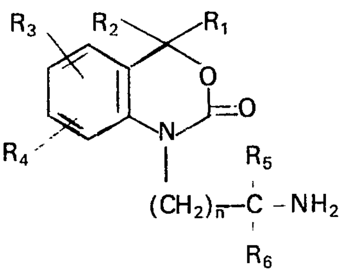

(III),

worin n und $R_2$–$R_6$ die obige Bedeutung haben, mit einer Verbindung der allgemeinen Formel

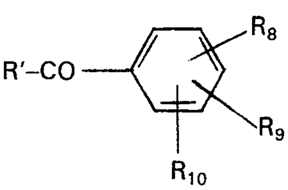

(IV),

worin $R_8$, $R_9$ und $R_{10}$ die obige Bedeutung haben und R' für eine der Gruppen

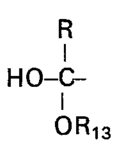

oder

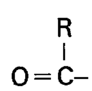

(Va) (Vb)

steht (R wie oben definiert und $R_{13}$ gleich $C_1$–$C_4$-Alkyl).

Als Reduktionsmittel für die reduktive Alkylierung werden komplexe Hydride, vorzugsweise Natriumborhydrid, Natriumcyanborhydrid, oder die katalytische Hydrierung, vorzugsweise mit den Katalysatoren Platin, Palladium oder Nickel, eingesetzt.

Beim Zusammengeben der Amine der Formel III und der Halbacetale bzw. Carbonverbindungen der Formel IV können sich ganz oder teilweise Schiffsche Basen der Formel

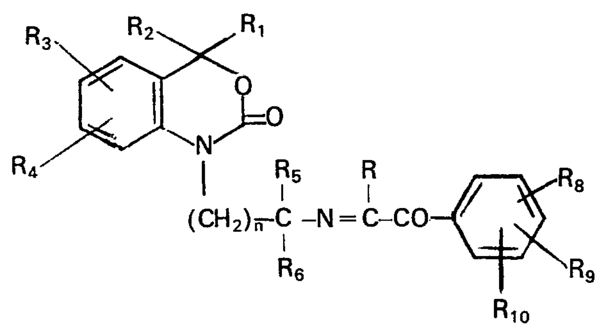

(VI)

als Zwischenstufe bilden. Die Reduktion mit den oben genannten Reduktionsmitteln erfolgt unabhängig davon, ob und in welchem Ausmass diese Schiffschen Basen entstehen.

3) Herstellung von Verbindungen der Formel I, in denen $R_9$ und/oder $R_8$ in der Bedeutung –OH stehen: Abspaltung der Schutzgruppe $R_{14}$ aus einer Verbindung der allgemeinen Formel

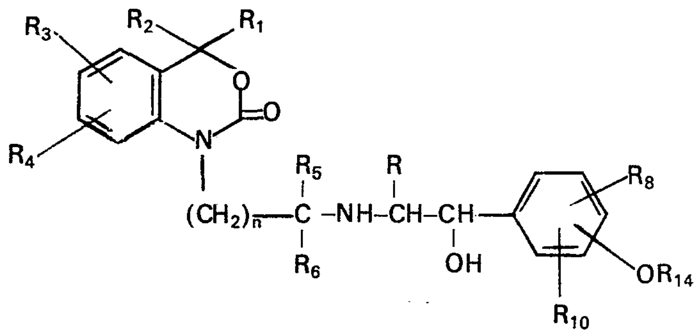

(VII)

in der n, R und $R_1$–$R_{10}$ die obige Bedeutung haben und $R_{14}$ für gegebenenfalls substituiertes Benzyl, CO–($C_1$–$C_4$-Alkyl) oder CO-Aryl steht.

Bei Verbindungen der Formel VII, in denen $R_8$ in der Bedeutung $OCH_2$Aryl oder OCOAlkyl steht, können dabei gleichzeitig auch diese Gruppen gespalten werden, so dass dann auch $R_8$ die Bedeutung OH-Gruppe annimmt.

Die nach diesem Verfahren erhaltenen Verbindungen entsprechen der allgemeinen Formel

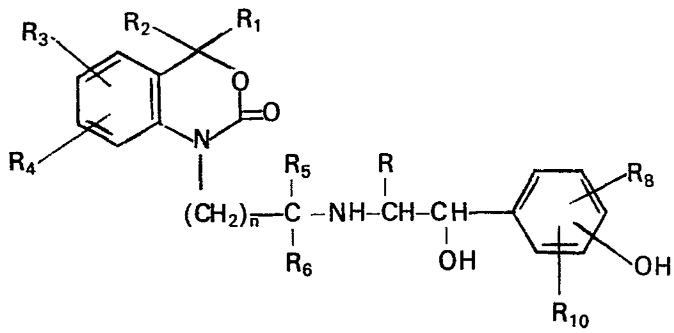

(VIII)

Die Abspaltung der Benzylgruppe erfolgt durch katalytische Hydrierung, vorzugsweise mit einem der Katalysatoren Platin, Palladium oder Nickel. Acylgruppen werden durch Verseifung mit verdünnten Säuren oder verdünnten Laugen, z.B. verdünnter Natronlauge, entfernt.

4) Herstellung von Verbindungen der Formel I, in denen $R_8$ in der Bedeutung $CONHR_{11}$ steht:

Umsetzung von Verbindungen der allgemeinen Formel

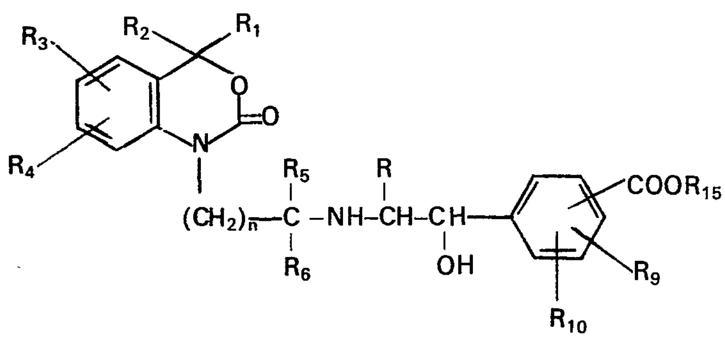

(IX),

worin n, R und $R_1$–$R_{10}$ die obige Bedeutung haben und $R_{15}$ einen $C_1$–$C_4$-Alkyl- oder Aralkylrest bedeutet, mit einem Amin der allgemeinen Formel

$H_2NR_{11}$ (X),

worin $R_{11}$ die obige Bedeutung hat.

Es enstehen Verbindungen der allgemeinen Formel

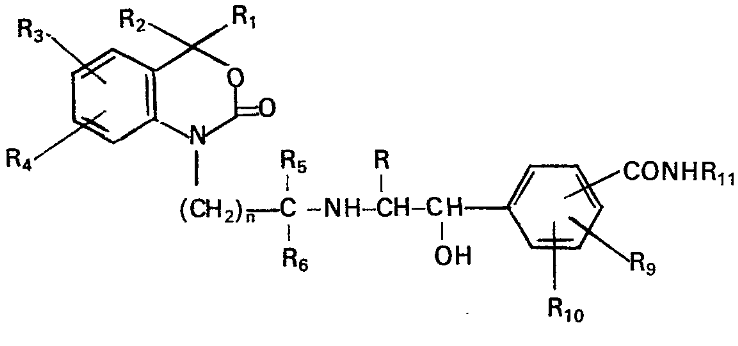

(XI)

Die nach den Verfahren 1) bis 4) erhaltenen Produkte werden gegebenenfalls anschliessend in diastereomeren Antipodenpaare oder in die einzelnen Enantiomeren aufgetrennt. Primär erhaltene Basen werden gewünschtenfalls in Säureadditionssalze, Säureadditionssalze in freie Basen umgewandelt.

Herstellung der Zwischenprodukte

Die Verbindungen der allgemeinen Formel XII:

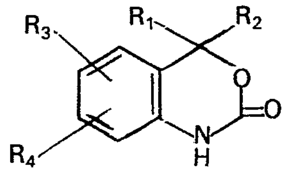

(XII)

sind aus der Literatur bekannt oder können nach den zur Herstellung der bekannten Verbindungen beschriebenen Verfahren hergestellt werden. Die Herstellung erfolgt ausgehend von den entsprechend substituierten Anthranilsäureestern (XIII) durch eine Umsetzung mit Grignard-Reagenz der allgemeinen Formel $R_1MgX$ (X gleich Chlor, Brom oder Jod):

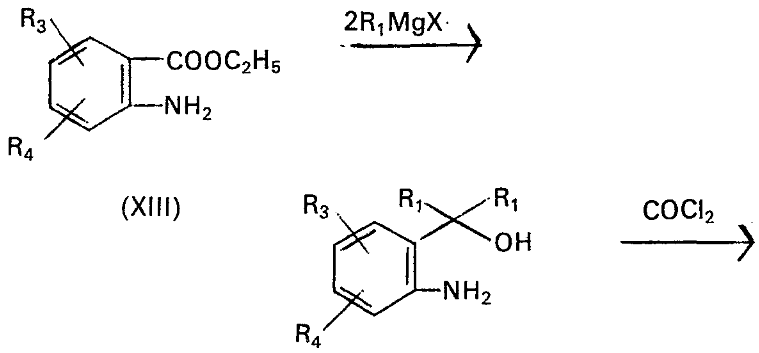


(XIII)

(XIV)

(XII)

zu denen Carbinolen der allgemeinen Formel XIV, die dann durch Umsetzung mit Phosgen oder Chlorameisensäureester in die 3,1-Benzoxazin-2-one der allgemeinen Formel (XII) überführt werden ($R_1$ und $R_2$ gleiche Alkylreste).

Zur Herstellung der 3,1-Benzoxazin-2-one der allgemeinen Formel

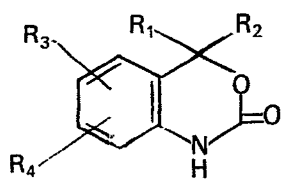

(XV)

($R_1$ und $R_2$ gleiche oder verschiedene Alkylreste) werden die o-Aminoacetophenonderivate der allgemeinen Formel (XVI) mit Grignard-Reagenz der Formel $R_2MgX$ umgesetzt und anschliessend mit Phosgen oder Chlorameisensäureester zu den 3,1-Benzoxazin-2-onen der allgemeinen Formel

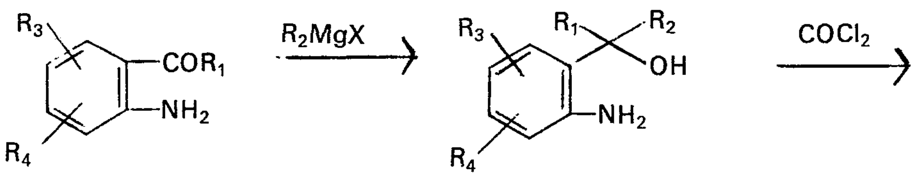


(XV)

(XVI)

Die Herstellung der Verbindungen der Formel XVII:

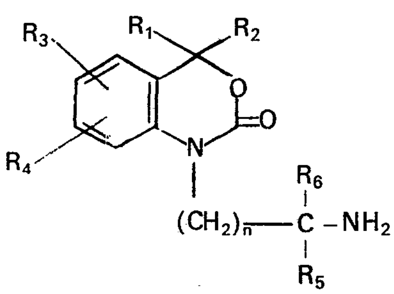

(XVII)

erfolgt durch Umsetzung der 3,1-Benzoxazin-2-one der allgemeinen Formel XII bzw. XV mit den Verbindungen der allgemeinen Formel XVIII in Gegenwart von Natriumhydrid und anschliessender Hydrolyse des Umsetzungsproduktes der allgemeinen Formel XIX:

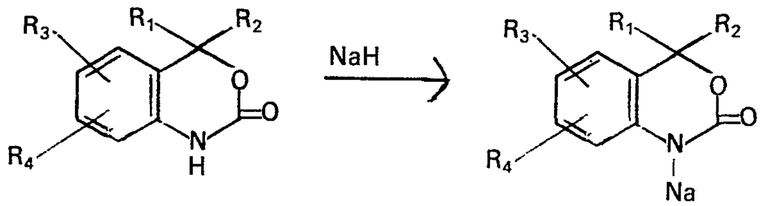


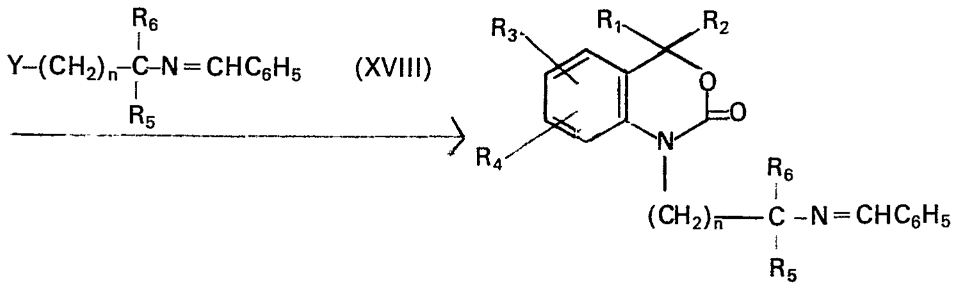

(XVIII)

(XIX)

$H_2O/H^+$ (XVII)

(Y: Cl, $OSO_2CH_3$, $OSO_2C_6H_4CH_3$)

Die Herstellung der Verbindungen der allgemeinen Formel II erfolgt ausgehend von den Verbindungen XVII durch Umsetzung mit Bromketonen der allgemeinen Formel

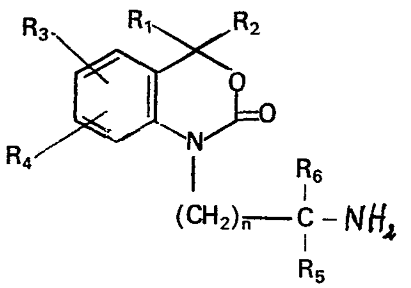

(XVII)

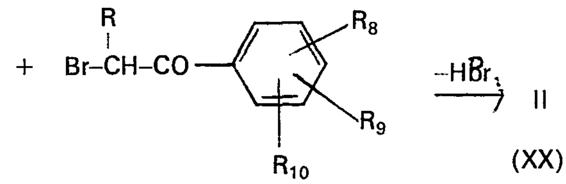


(XX)

Als Bindemittel für den bei der Reaktion entstehenden Bromwasserstoff wird überschüssiges Amin der allgemeinen Formel XVII oder Alkali, wie z.B. Soda oder Kaliumbicarbonat, verwendet.

Die Aminoketone der allgemeinen Formeln XXI, XXII und XXIII:

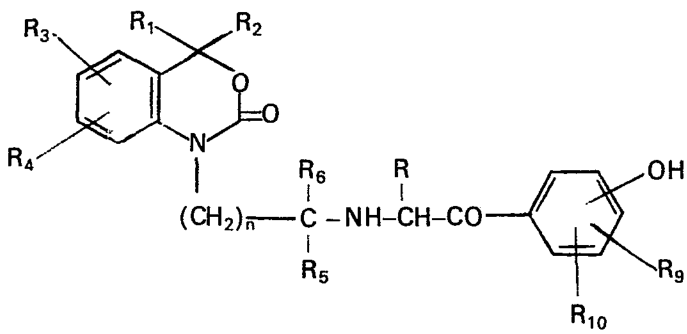

(XXI),

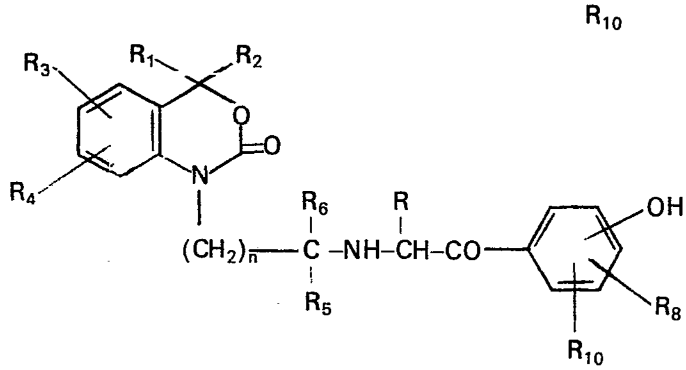

(XXII),

und

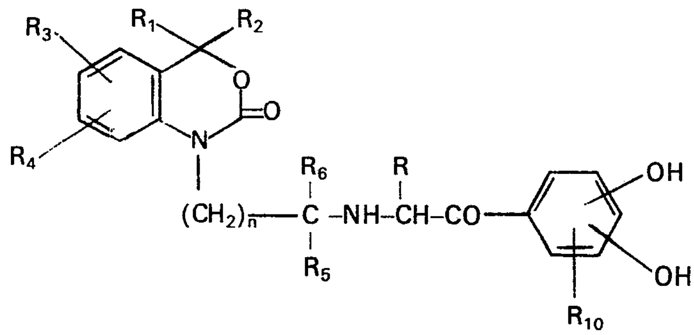

(XXIII)

können hergestellt werden aus den Verbindungen der allgemeinen Formeln XXIV, XXV und XXVI:

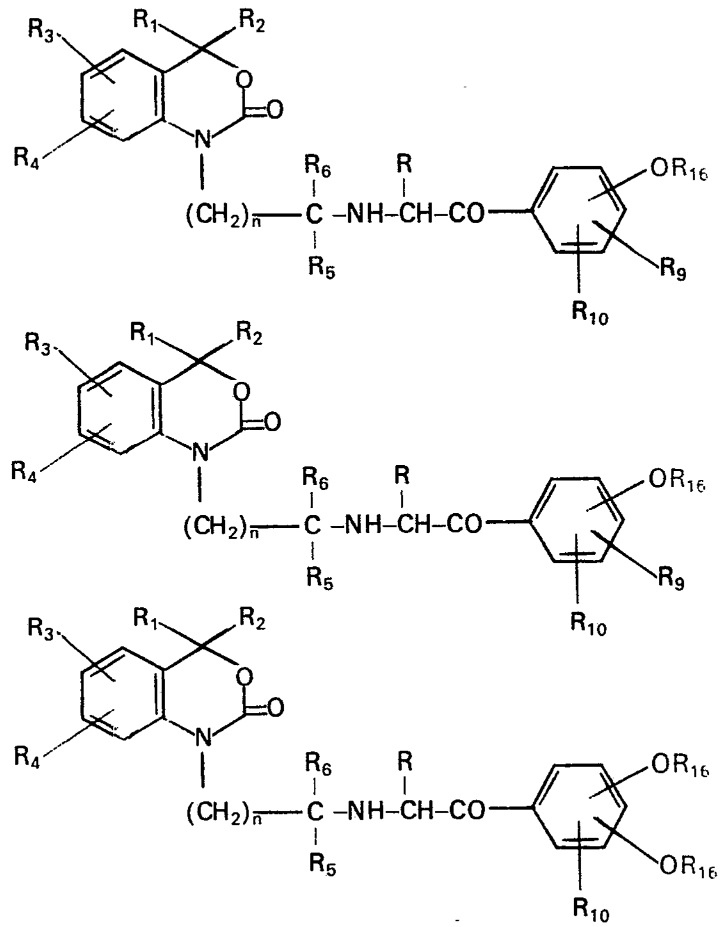

(XXIV)

(XXV)

(XXVI)

$R_{16}$: $CH_2C_6H_5$, CO–($C_1$–$C_4$)-Alkyl durch Abspaltung der Schutzgruppe $R_{16}$.

Die Abspaltung der Schutzgruppe $R_{16}$ erfolgt, wenn $R_{16}$ $CH_2C_6H_5$ bedeutet, durch eine katalytische Hydrierung mit Platin, Palladium oder Raney-Nickel als Katalysator. Wenn $R_{16}$ die CO–($C_1$–$C_4$)-Alkylgruppe bedeutet, erfolgt die Abspaltung der Schutzgruppe durch eine Verseifung, besonders in Gegenwart einer Säure, wie z.B. verdünnte Salzsäure.

Die erfindungsgemässen Verbindungen sind wertvolle Arzneistoffe und Zwischenprodukte für die Herstellung von Arzneistoffen. Hervorzuheben ist die blutdrucksenkende Wirkung sowie die sehr wirksame und selektive Tokolyse. Weiterhin sind die neuen Verbindungen als Broncholytika, Gefässerweiterer und Herzmittel verwendbar. Wichtig ist auch die gute Wirkungsdauer.

Für die Anwendung werden die erfindungsgemässen Wirkstoffe mit den in der galenischen Pharmazie üblichen Hilfsstoffen zu gebräuchlichen Arzneimittelformen verarbeitet, z.B. zu Tabletten, Dragées, Kapseln, Tinkturen, Injektionslösungen, Suppositorien, Inhalationspulvern, Dosieraerosolen.

Die Dosierung der neuen Verbindungen ist in Abhängigkeit von der Indikation, der Applikationsart und der verwendeten Substanz sowie dem Körpergewicht der zu behandelnden Person unterschiedlich. Für die Verwendung als Blutdrucksenker beim Erwachsenen liegt die orale Dosis zwischen 5 und 2000 mg, vorzugsweise

zwischen 20 und 1000 mg. Als Tokolytika werden Tabletten mit 0,1 bis 50 mg, vorzugsweise mit 0,5 bis 20 mg oder Ampullen mit 0,1 bis 10 mg, vorzugsweise 0,2 bis 2 mg Wirkstoff verwendet.

Die blutdrucksenkende Wirkung der erfindungsgemässen Verbindungen wurde nach der im folgenden beschriebenen Methode ermittelt:

An wachen, genetisch hypertonen männlichen Ratten (SH Ratten des Stammes OKAMOTO und AOKI) wurde der Blutdruck (Mitteldruck) über einen nach dem Verfahren von WEEKS chronisch in die Aorta implantierten Katheter mit Stathampressure-transducern auf einem Kompensationsschreiber registriert. Die Herzfrequenz wurde aus der Anzahl der Pulswellen errechnet. Den frei beweglichen und mit der Methode vertrauten Tieren wurde nach einer einstündigen Vorperiode die Versuchssubstanz oral verabreicht. Blutdruck und Herzfrequenz wurden danach 6 Stunden lang registriert. War nach Ablauf dieser Zeit der Ausgangswert für Blutdruck und/oder Herzfrequenz noch nicht annähernd wieder erreicht, wurden am nächsten Tag weitere Messungen vorgenommen.

Die Testsubstanz wurde als Suspension der Lösung in 1%iger wässriger Tylose-Lösung oral mit der Schlundsonde appliziert. Die applizierten Dosen betrugen 10 mg/kg. Literatur: OKAMOTO, K. und K. AOKI: Jap. Circul. J. 87, 2821 (1963) WEEKS, J.R. und J.A. JONES: Proc. Soc. Exp. Biol. Med. 104, 646 (1960).

Nach dieser Methode wurde beispielsweise für 1-(3-Methylcarbamoyl-4-hydroxyphenyl)-2-{1,1-dimethyl-3-[1-(4,4-dimethyl-3,1-benzoxazin- 2-onyl)]-propylamino}-ethanolmethansulfonat die Wirkung auf Blutdruck (RR; Werte in mbar) und Herzfrequenz (HF; Schläge pro Minute) an genetischen Hochdruckratten bei mehrmaliger oraler Gabe bestimmt:

| | Vorwert | 6-Stunden-Wert | |
|---|---|---|---|
| 1. Tag Gabe | 274 | 248 | RR |
| 10 mg/kg | 389 | 390 | HF |
| 2. Tag Gabe | 238 | 226 | RR |
| 10 mg/kg | 384 | 345 | HF |
| 3. Tag Gabe | 214 | 202 | RR |
| 10 mg/kg | 320 | 360 | HF |
| 4. Tag 1 ml/kg | 217 | 228 | RR |
| NaCl 0,9% | 336 | 328 | HF |
| 5. Tag 1 ml/kg | 252 | 266 | RR |
| NaCl 0,9% | 350 | 325 | HF |

Wie die Tabelle zeigt, sinkt an den ersten drei Tagen der Blutdruck nach jeder Gabe unter den Vorwert. Auch an den darauffolgenden Tagen steigt der Blutdruck nur langsam wieder bis in die Nähe des Ausgangswertes an. Die $LD_{50}$ der o.g. Verbindung beträgt >1500 mg/kg (p.o. an der Ratte).

Die folgenden Angaben für 1-(3-Methansulfonamido -4- hydroxyphenyl) -2- {1,1-dimethyl -3- [1-(4-methyl - 3,1-benzoxazin- 2- onyl)] -propylamino}-ethanol illustrieren die tokolytische Wirkung der erfindungsgemässen Verbindungen.

An Ratten in Urethannarkose bewirkt die obige Verbindung in einer Dosis von 0,035 µg/kg i.v. bei 50% der Versuchstiere eine Verringerung der Amplitude der Uteruskontraktionen von mehr als 10%, Dauer 8 Minuten. Bei dieser Dosis wird eine Steigerung der Herzfrequenz um 2 Schläge/Minute beobachtet, Dauer 6 Minuten. Zusätzlich wurde die Frequenz der Uteruskontraktionen in die Beurteilung einbezogen. Dazu wurde das Produkt aus Amplitudenhöhe und Frequenz für Perioden von 5 Minuten Dauer ermittelt; Ausgangswert gleich 100%. 0,09 µg/kg i.v. bewirkten eine Verminderung dieses Wertes um 50%, Dauer 14 Minuten. Diese Dosis verursacht eine Steigerung der Herzfrequenz um 2 Schläge/Minute, Dauer 6 Minuten.

Die Formulierung der erfindungsgemässen Wirkstoffe kann entsprechend den folgenden Beispielen vorgenommen werden:

A. Kapseln

Zusammensetzung:

| | |
|---|---|
| 1-(3-Methylcarbamoyl-4-hydroxyphenyl)-2-{1,1-dimethyl-3-[1-(4,4-dimethyl-3,1-benzoxazin-2-onyl)]-propylamino}ethanolmethansulfonat | 100 mg |
| Maisstärke | 300 mg |
| | 400 mg |

Die Bestandteile werden entsprechend dem angegebenen Verhältnis gut vermischt und in 400 mg-Portionen in Gelatine-Steckkapseln abgefüllt.

B. Tabletten

Zusammensetzung:

| | |
|---|---|
| 1-(3-Methansulfonamido-4-hydroxyphenyl)-2-{1,1-dimethyl-3-[1-(4-methyl-3,1-benzoxazin-2-onyl)]-propylamino}-ethanolformiat | 2 mg |
| kolloidale Kieselsäure | 10 mg |
| Milchzucker | 116 mg |
| Kartoffelstärke | 60 mg |
| Polyvinylpyrrolidon | 6 mg |
| Na-Celluloseglykolat | 4 mg |
| Magnesiumstearat | 2 mg |
| | 200 mg |

Die Bestandteile werden in üblicher Weise zu Tabletten von 200 mg verarbeitet, die jeweils 2 mg Wirkstoff enthalten. Anstelle der angegebenen Verbindung können auch andere erfindungsgemässe Verbindungen verwendet werden.

C. Ampullen
Zusammensetzung der Lösung:

| | | |
|---|---|---|
| Wirkstoff gemäss der Erfindung | | 2 mg |
| Sorbit | | 40 mg |
| destill. Wasser | ad | 10 ml |

D. Inhalationspulver in Kapseln

Pro Hartgelatine-Steckkapsel wird eine Mischung von 0,5 mg eines Wirkstoffs gemäss der Erfindung, z.B. 1-(3-Methansulfonamido-4-hydroxyphenyl)-2-{1,1-dimethyl-3-[1-(4,4-dimethyl-6-hydroxy-3,1-benzoxazin-2-onyl)]-propylamino}-ethanol-hydrochlorid und 19,5 mg Lactose mit einem Teilchendurchmesser zwischen 0,5 und 7 µm eingefüllt.

Die erfindungsgemässen Wirkstoffe können auch mit bekannten Wirkstoffen kombiniert werden; für die broncholytische Anwendung z.B. mit Theophyllinen, Parasympatholytika (z.B. Ipratropiumbromid), Sekretolytika (z.B. Bromhexin), muskulotropen Spasmolytika (z.B. Papaversin), Corticoiden, Antiallergica.

Bei den Uterusrelaxantien sind u.a. Kombinationen mit Corticoiden möglich und bei der Blutdrucksenkung mit Saluretika und anderen Antihypertonika.

Die erfindungsgemässen Verfahren sind in den nachstehenden Beispielen näher erläutert:

Beispiele für das Verfahren 1

Beispiel 1

1-(3-Methylcarbamoyl-4-hydroxyphenyl)-2-{1,1-dimethyl-3-[1-(4,4-dimethyl-3,1-benzoxazin-2-onyl)]-propylamino}ethanol

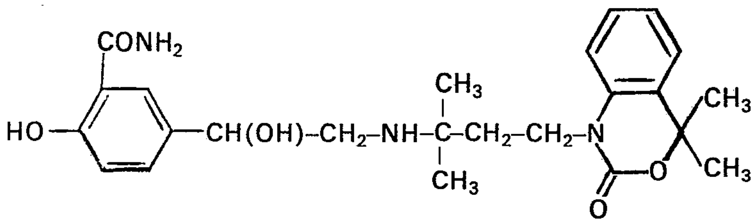

Eine Mischung von 2,6 g 3-Carbamoyl-4-hydroxy-α-bromacetophenon und 2,6 g 1,1-Dimethyl-3-[1-(4,4-dimethyl-3,1-benzoxazin-2-onyl)]propylamin werden in 50 ml Essigester 25 Minuten unter Rückfluss gekocht. Die noch warme Lösung wird von dem ausgefallenen Amin-Hydrobromid abgesaugt, das Filtrat abgekühlt, mit 10 ml Äthanol verdünnt und bei 0 °C mit 0,2 g Natriumborhydrid versetzt. Die bei einer Temperatur von 0–10 °C gehaltene Lösung wird 2 Stunden gerührt und dann mit 8 ml 50%iger Essigsäure angesäuert. Nach dem Abdestillieren des Lösungsmittels im Vakuum wird der Rückstand mit einer Lösung von 1,4 g Kaliumcarbonat in 7 ml Wasser versetzt und das Gemisch dreimal mit Essigester ausgeschüttelt. Die Essigesterphase wird über Natriumsulfat getrocknet und soweit eingeengt, bis die Titelverbindung auszukristallisieren beginnt. Es werden 1,1 g Substanz erhalten, die nach dem Umkristallisieren einen Schmelzpunkt von 197 °C zeigen. Durch Zugabe von Methansulfonsäure wird in Acetonitril das Methansulfonat (Fp. 138 °C) erhalten.

Beispiel 2

1-(3-Methansulfonamido-4-hydroxyphenyl)-2-{1,1- dimethyl-3- [1-(4,4-dimethyl-3,1-benzoxazin-2-onyl)]-propylamino}ethanol

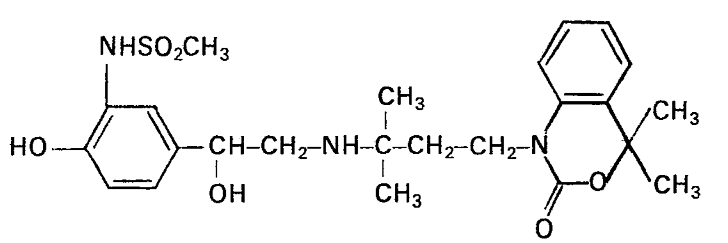

Eine Mischung von 4,0 g 3-Methansulfonamido-4-benzyloxy-α-bromacetophenon und 5,2 g 1,1-Dimethyl-3-[1-(4,4-dimethyl-3,1-benzoxazin-2-onyl)]-propylamin werden in 100 ml Essigester 30 Minuten unter Rückfluss gekocht. Nach dem Abkühlen wird das ausgefallene Amin-Hydrobromid abgesaugt, das Filtrat eingeengt. 5,5 g des Reaktionsprodukts (als Hydrochlorid Fp. 205 °C) werden in 120 ml Methanol gelöst und, zur Entfernung der Benzylgruppe, bei Raumtemperatur und Normaldruck mit Pd/C bis zur Aufnahme von 230 ml Wasserstoff hydriert. Nach dem Abtrennen des Katalysators wird das Methanol unter Vakuum vollständig abdestilliert und das gebildete Keton in Acetonitril gelöst. Durch Zugabe von ätherischer Salzsäure wird das Hydrochlorid (Fp. 257 °C) erhalten. 2 g dieser Substanz werden in das Hydrochlorid (Fp. 257 °C) erhalten. 2 g dieser Substanz werden in 200 ml Methanol bei Raumtemperatur und Normaldruck mit 0,2 g Platinoxid als Katalysator bis zur Aufnahme von 84 ml Wasserstoff hydriert. Das entstandene Produkt ist die Titelverbindung, die als Base (Fp. 192 °C) in einer Ausbeute von 90% d.Th. isoliert wird.

Nach Verfahren 1) werden entsprechend erhalten:

| Beispiel | Verbindung | Kenndaten |
|---|---|---|
| 3 | 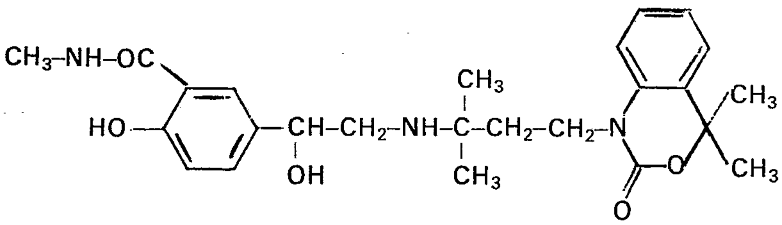 | Fp. Base: 195 °C<br>Fp. Salz: 204 °C<br>Methansulfonat |
| 4 | 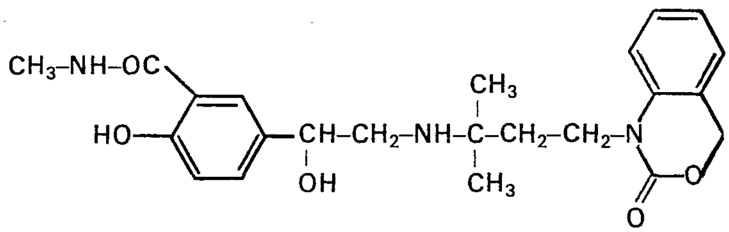 | Fp. Salz: 170 °C<br>Formiat |
| 5 | 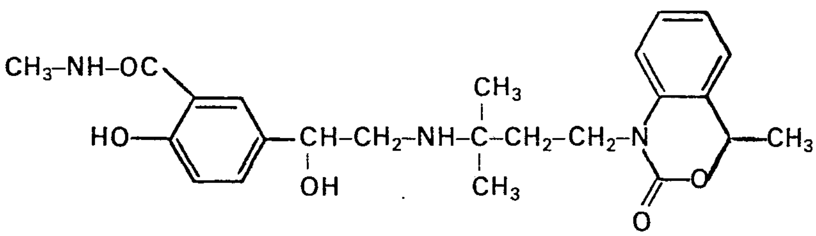 | Fp. Salz: 143 °C<br>Formiat |
| 6 | 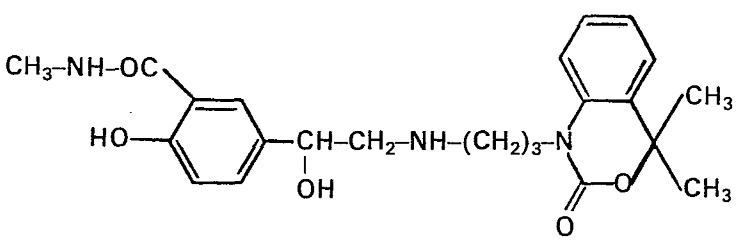 | Fp. Salz: 169 °C<br>Formiat |
| 7 | 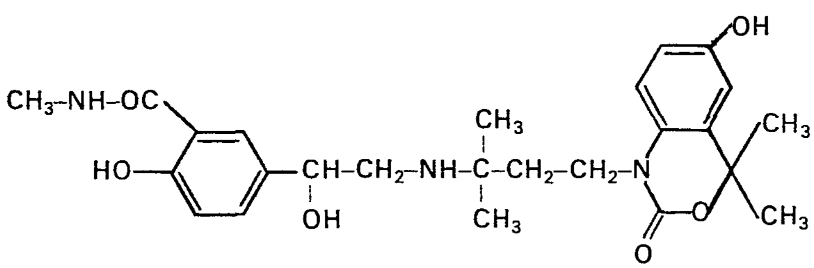 | Fp. Base: 176 °C<br>Fp. Salz: 181 °C<br>Formiat |
| 8 | 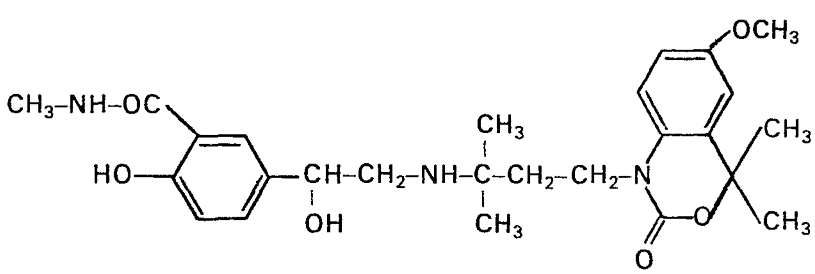 | Fp. Salz: 210 °C<br>Hydrochlorid |

(Fortsetzung)

| Beispiel | Verbindung | Kenndaten |
|---|---|---|
| 9 | 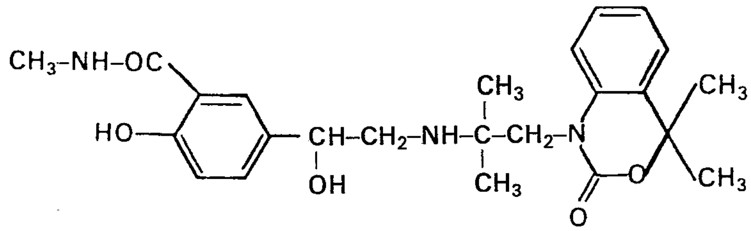 | Fp. Salz: 165 °C<br>p-Aminobenzoat |
| 10 | 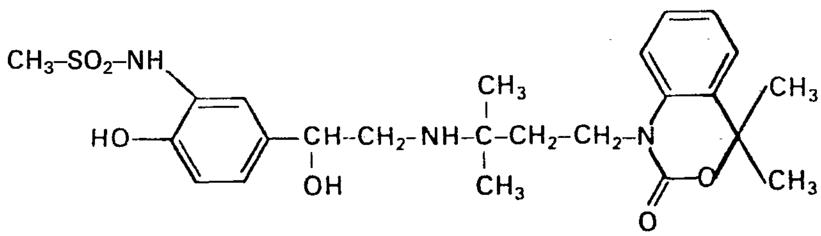 | Fp. Salz: 164 °C<br>Formiat |
| 11 | 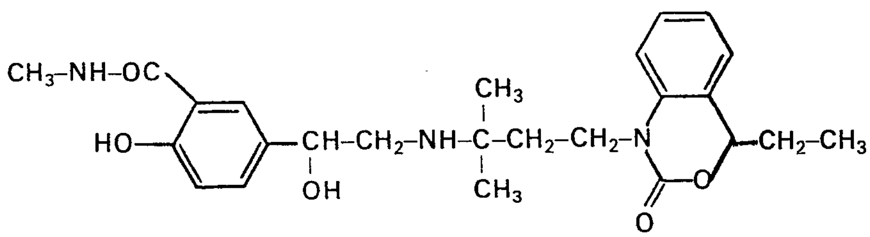 | Fp. Base: 163 °C<br>Fp. Salz: 181 °C<br>Hydrochlorid |
| 12 | 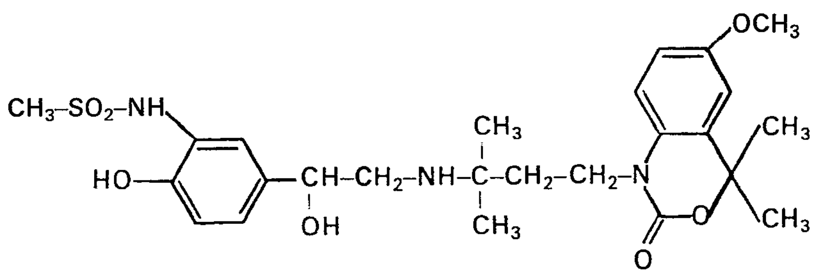 | Fp. Base: 181 °C<br>Fp. Salz: 183 °C<br>Hydrochlorid |
| 13 | 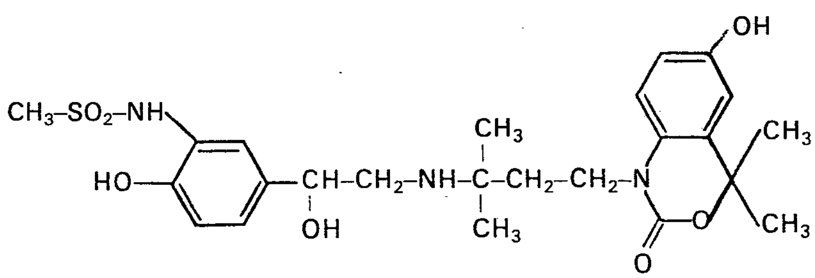 | Fp. Salz: 211 °C<br>Hydrochlorid |
| 14 | 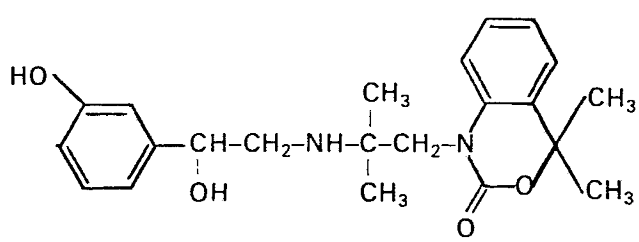 | Fp. Base: 140 °C<br>Fp. Salz: 166 °C<br>Fumarat |

(Fortsetzung)

| Beispiel | Verbindung | Kenndaten |
|---|---|---|
| 15 | 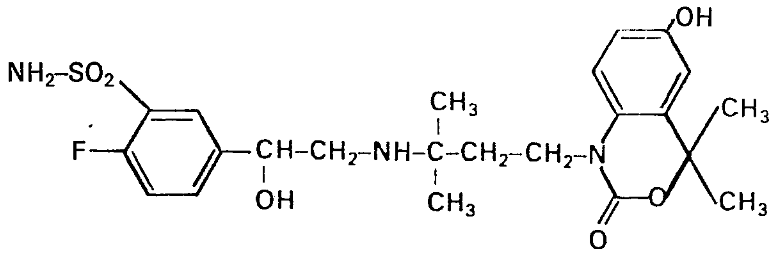 | Fp. Salz: 192 °C<br>Fumarat |
| 16 | 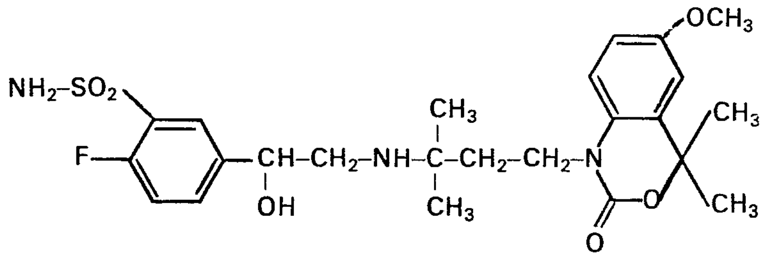 | Fp. Salz: 197 °C<br>Fumarat |
| 17 | 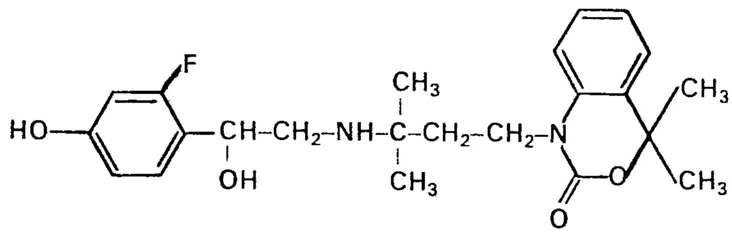 | Fp. Salz: 194 °C<br>Methansulfonat |
| 18 | 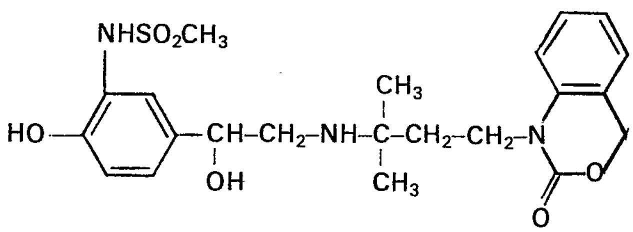 | Fp. Base: 200 °C<br>Fp. Salz: 180 °C<br>Formiat |
| 19 | 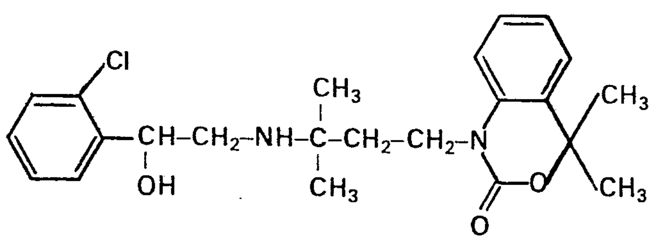 | Fp. Salz: 251 °C<br>Hydrochlorid |
| 20 | 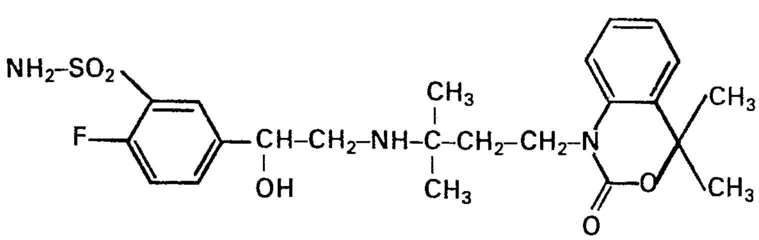 | Fp. Salz: 235 °C<br>Fumarat |

(Fortsetzung)

| Beispiel | Verbindung | Kenndaten |
|---|---|---|
| 21 | 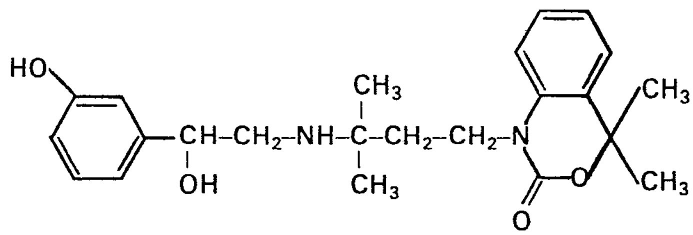 | Fp. Salz: 180 °C Formiat |
| 22 | 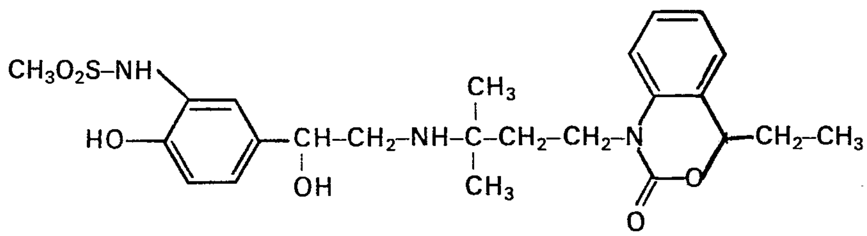 | Fp. Salz: 182 °C Hydrochlorid |
| 23 | 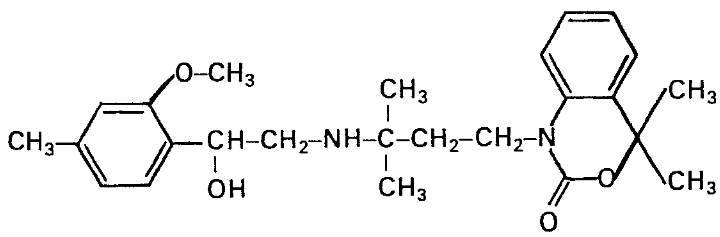 | Fp. Salz: 223 °C Hydrochlorid |
| 24 | 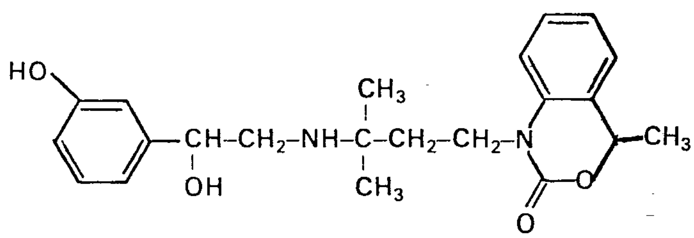 | Fp. Salz: 153 °C Formiat |
| 25 | 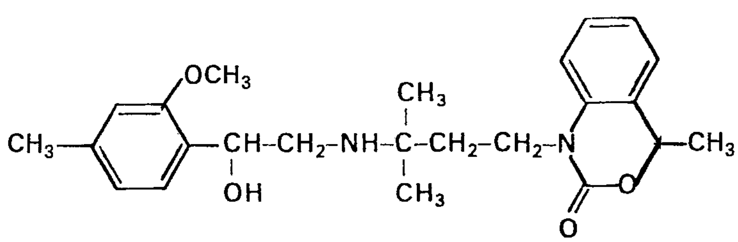 | Fp. Salz: 190 °C Hydrochlorid |
| 26 | 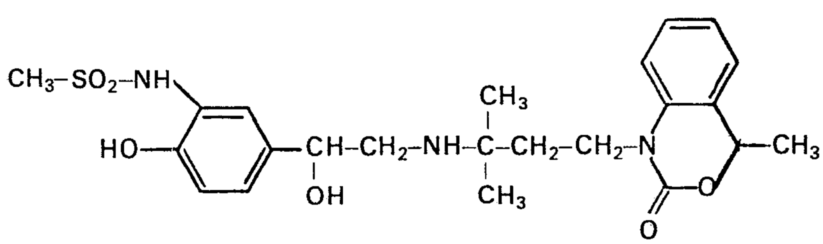 | Fp. Salz: 192 °C Formiat |

(Fortsetzung)

| Beispiel | Verbindung | Kenndaten |
|---|---|---|
| 27 | 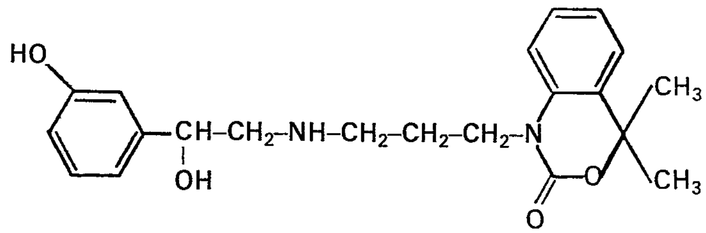 | Fp. Salz: 147 °C Formiat |
| 28 | 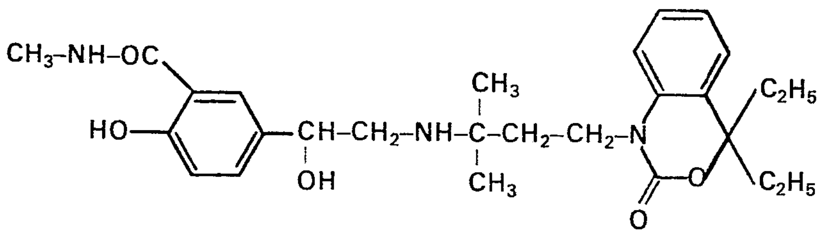 | Fp. Salz: 214 °C Methansulfonat |
| 29 | 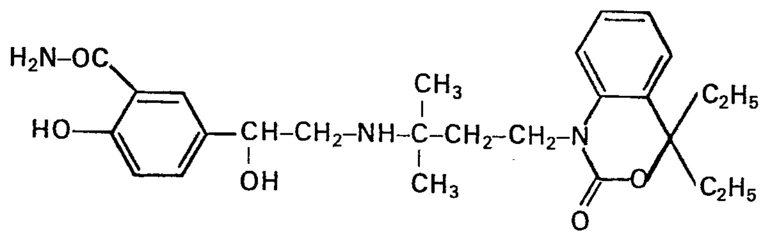 | Fp. Base: 183 °C Fp. Salz: 208 °C Hydrochlorid |
| 30 | 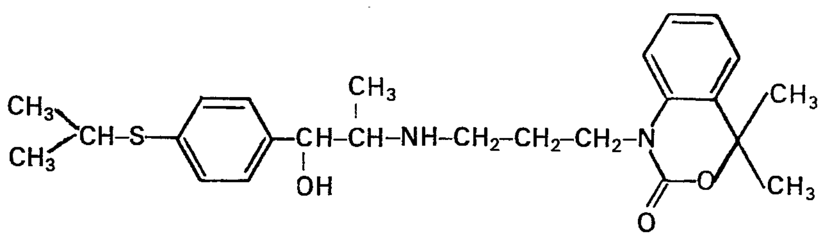 | Fp. Salz: 153 °C Hydrochlorid |
| 31 | 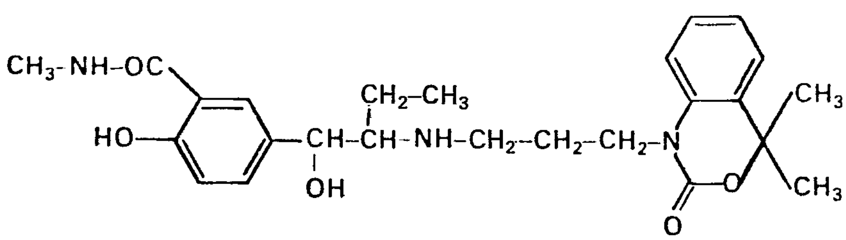 | Fp. Salz: 165 °C Methansulfonat |
| 32 | 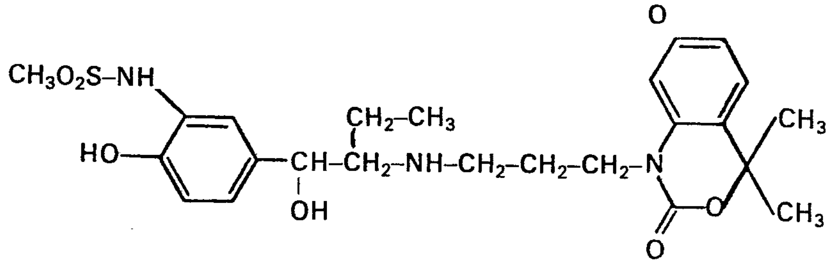 | Fp. Salz: 183 °C Succinat |
| 33 | 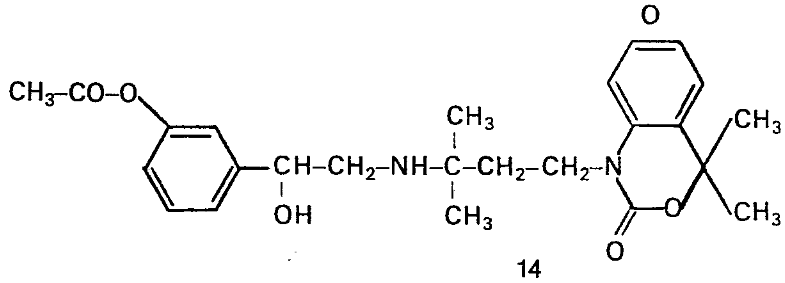 | Fp. Salz: 102 °C Formiat |

(Fortsetzung)

| Bei-spiel | Verbindung | Kenndaten |
|---|---|---|
| 34 | 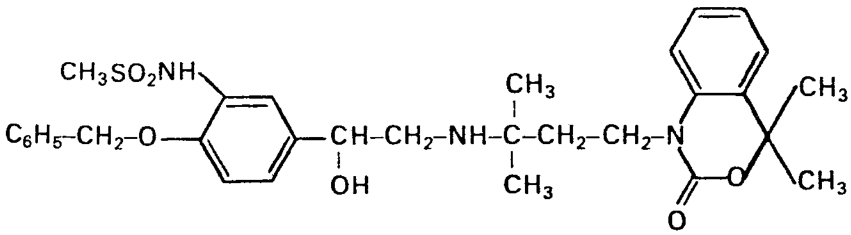 | Fp. Salz: 162 °C Formiat |
| 35 | 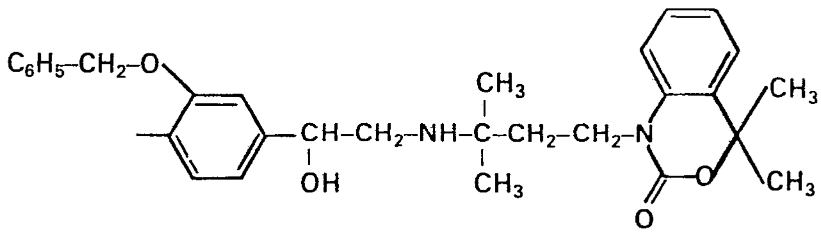 | Fp. Salz: 167 °C Fumarat |
| 36 | 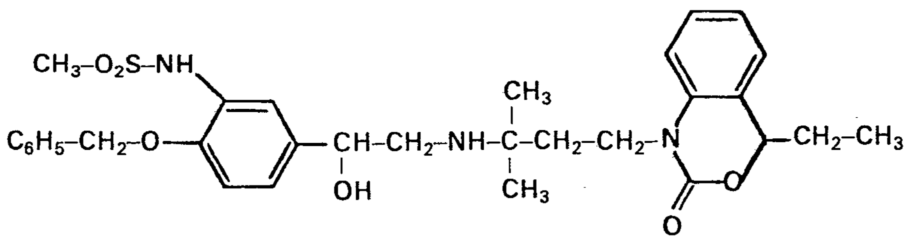 | Fp. Salz: 137 °C Maleinat |
| 37 | 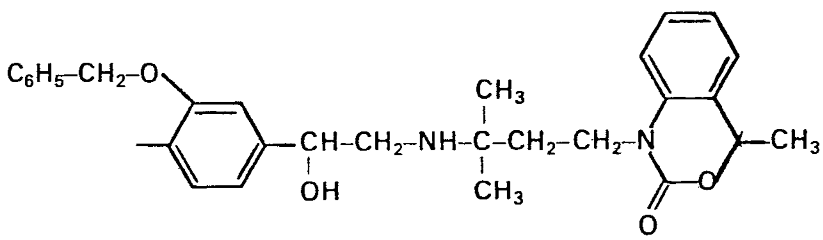 | Fp. Salz: 157 °C Fumarat |
| 38 | 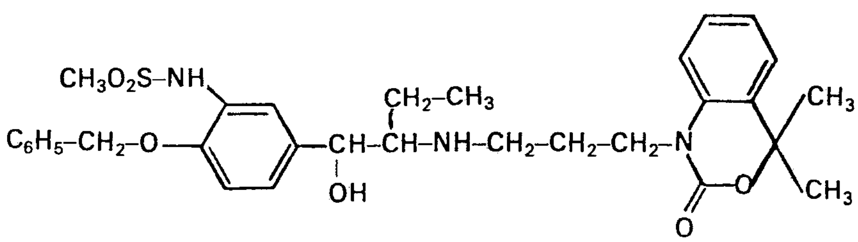 | amorph |
| 39 | 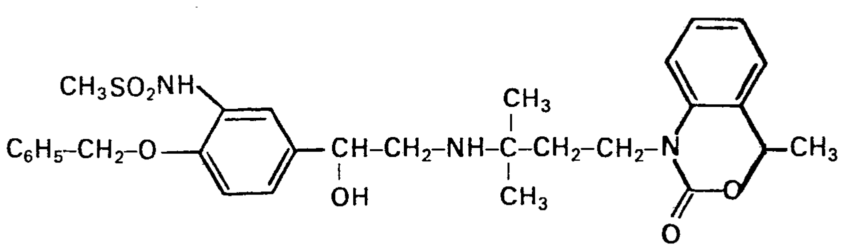 | Fp. Salz: 166 °C Maleinat |

(Fortsetzung)

| Beispiel | Verbindung | Kenndaten |
|---|---|---|
| 40 | 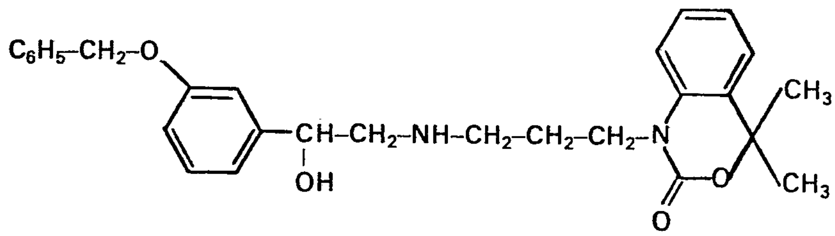 | Fp. Salz: 142 °C Hydrochlorid |
| 41 | 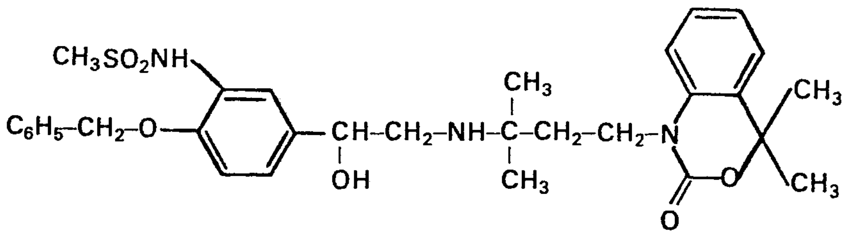 | amorph |
| 42 | 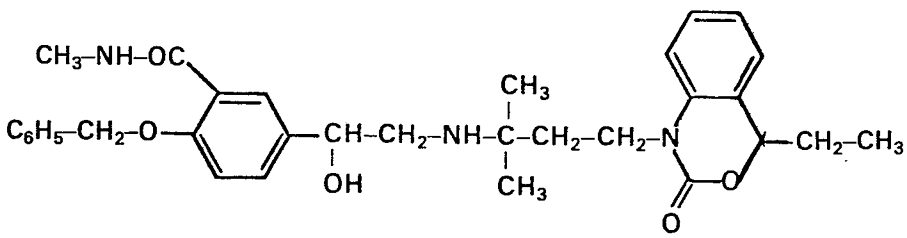 | amorph |
| 43 | 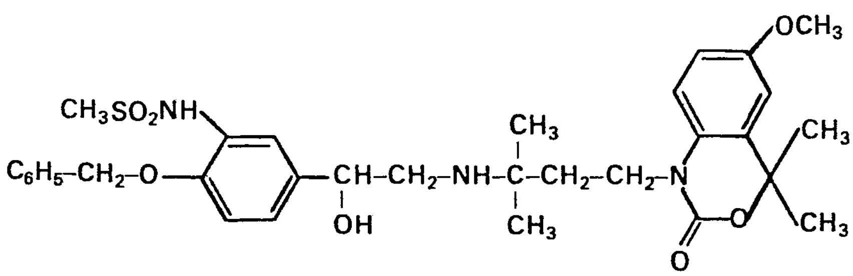 | amorph |
| 44 | 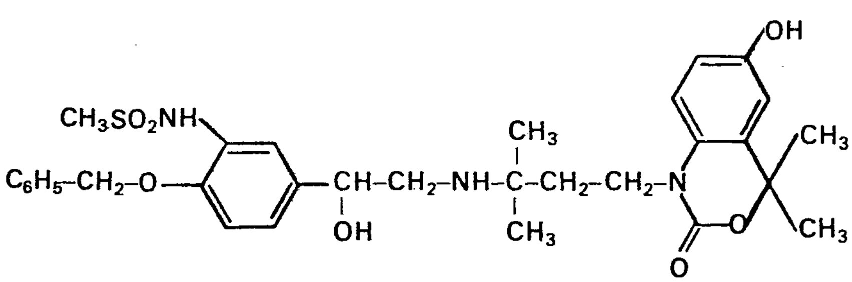 | amorph |
| 45 | 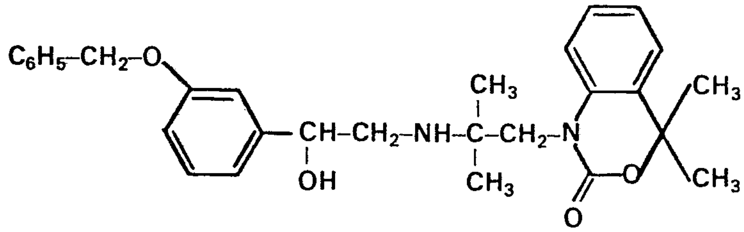 | Fp. Salz: 173 °C Fumarat |

(Fortsetzung)

| Bei-spiel | Verbindung | Kenndaten |
|---|---|---|
| 46 | 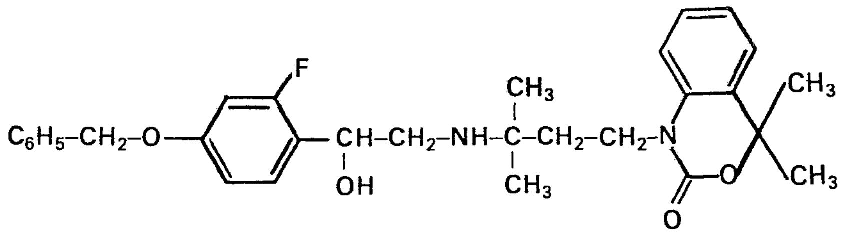 | Fp. Salz: 192 °C Methansulfonat |
| 47 | 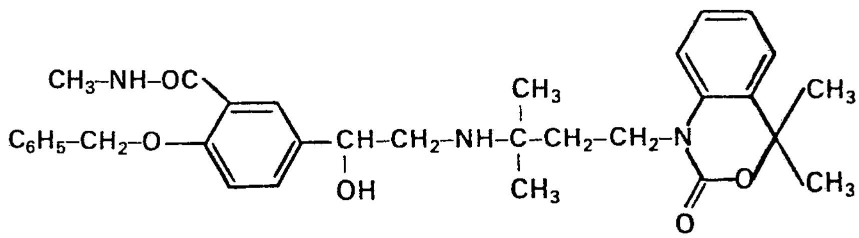 | Fp. Salz: 183 °C Methansulfonat |
| 48 | 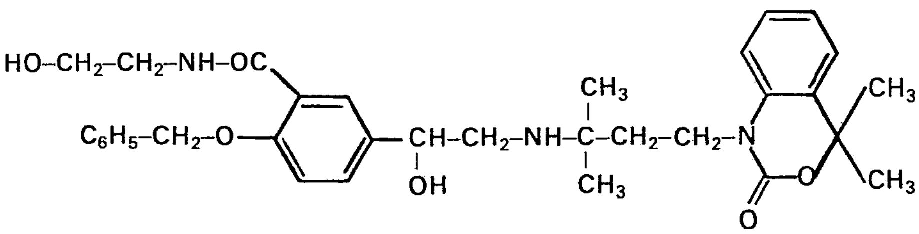 | Fp. Salz: 170 °C Maleinat |
| 49 | 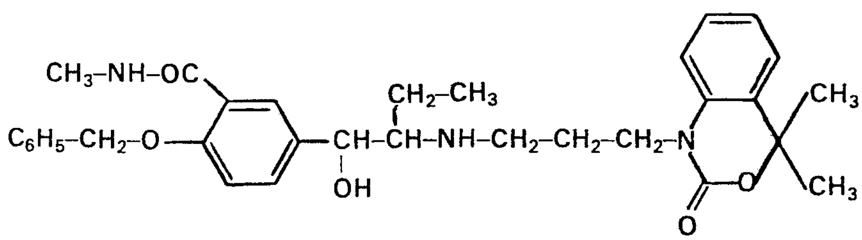 | Fp. Salz: 142 °C Maleinat |
| 50 | 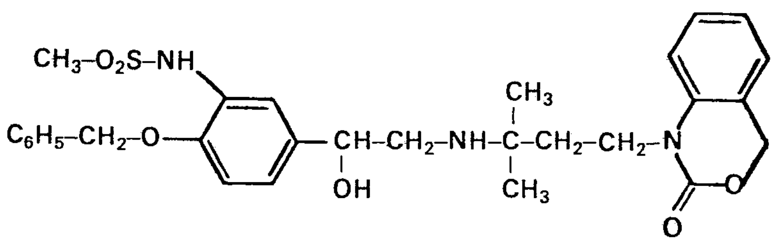 | Fp. Salz: 189 °C Maleinat |
| 51 | 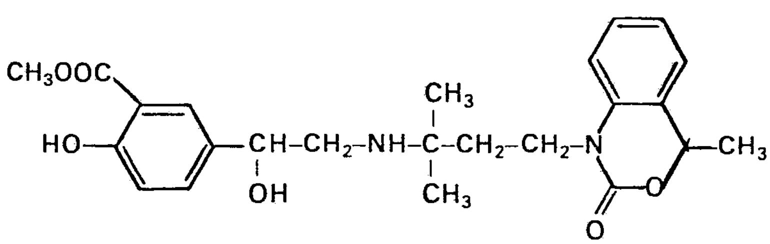 | Fp. Salz: 155 °C p-Aminobenzoat |

(Fortsetzung)

| Beispiel | Verbindung | Kenndaten |
|---|---|---|
| 52 | 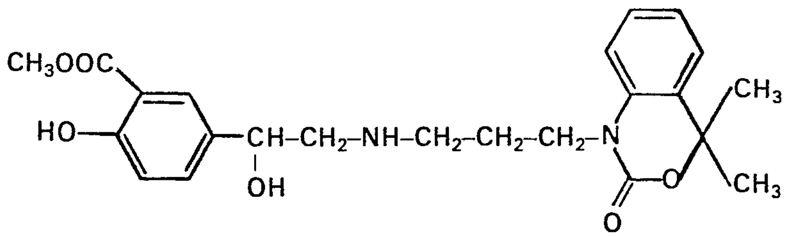 | Fp. Salz: 191 °C<br>Hydrochlorid |
| 53 | 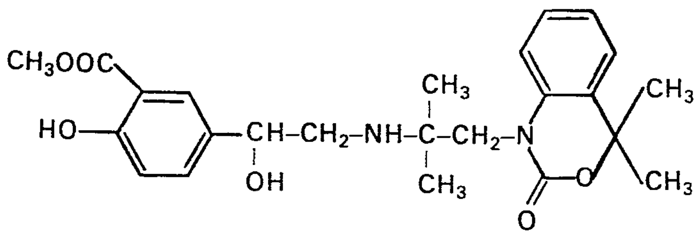 | Fp. Salz: 153 °C<br>p-Aminobenzoat |
| 54 | 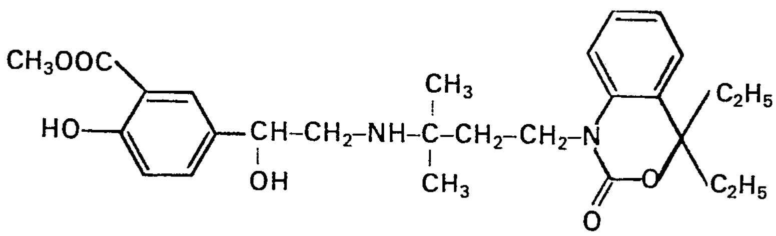 | Fp. Salz: 89 °C<br>Maleinat |
| 55 | 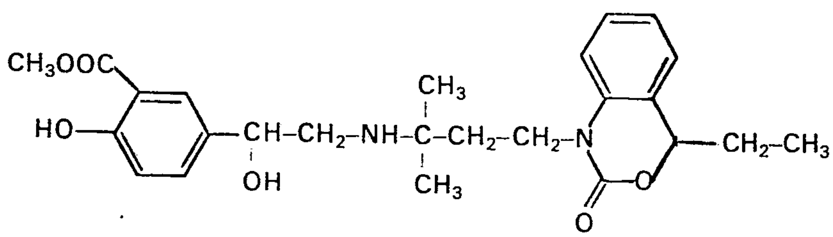 | Fp. Salz: 120 °C<br>Maleinat |
| 56 | 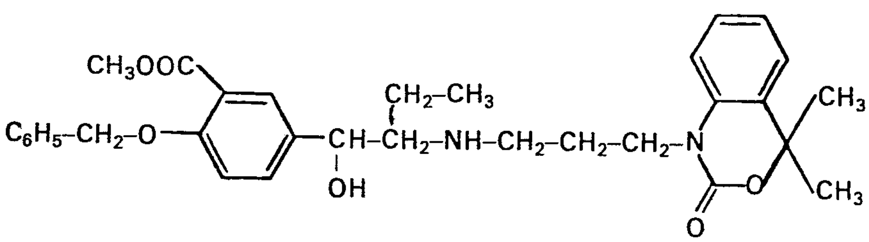 | Fp. Salz: 142 °C<br>Maleinat |
| 57 | 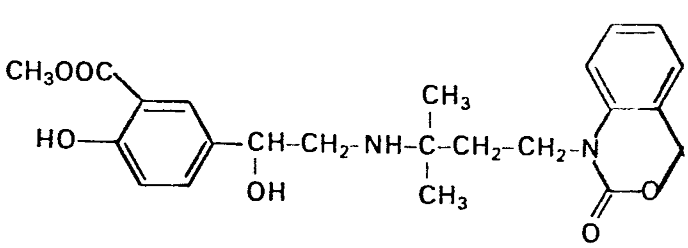 | Fp. Salz: 188 °C<br>p-Aminobenzoat |

(Fortsetzung)

| Beispiel | Verbindung | Kenndaten |
|---|---|---|
| 58 | 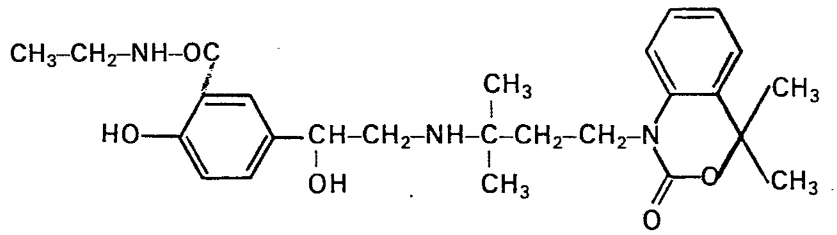 | Fp. Salz: 200 °C<br>Methansulfonat |
| 59 | 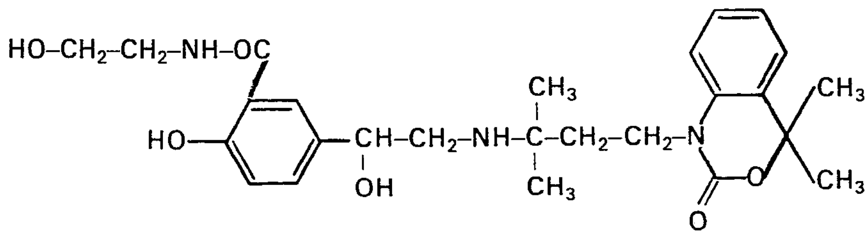 | Fp. Base: 161 °C<br>Fp. Salz: 181 °C<br>Hydrochlorid |
| 60 | 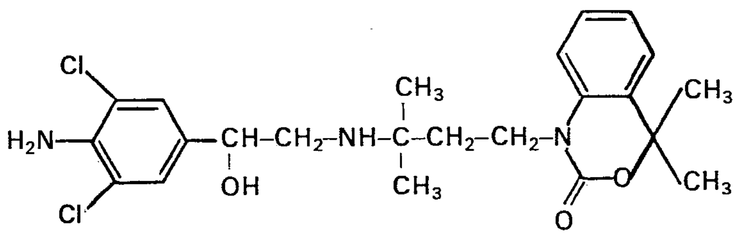 | Fp. Salz: 170 °C<br>Maleinat |
| 61 | 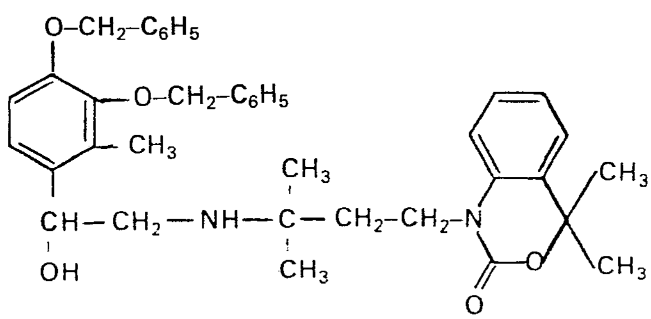 | Fp. Salz: 212 °C<br>Hydrochlorid |
| 62 | 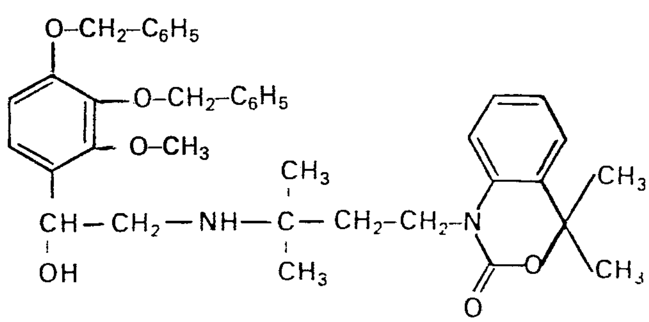 | Fp. Salz: 195 °C<br>Hydrochlorid |

Beispiele für das Verfahren 2

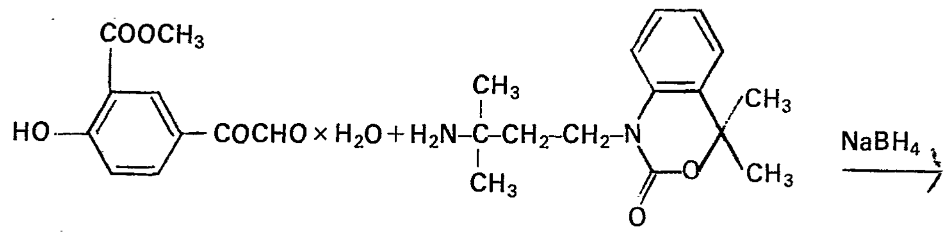


Beispiel 63
1-(3-Methoxycarbonyl-4-hydroxyphenyl)-2-{1,1-dimethyl-3-[1-(4,4-dimethyl-3,1-benzoxazin-2-onyl)]-propylamino}-ethanol

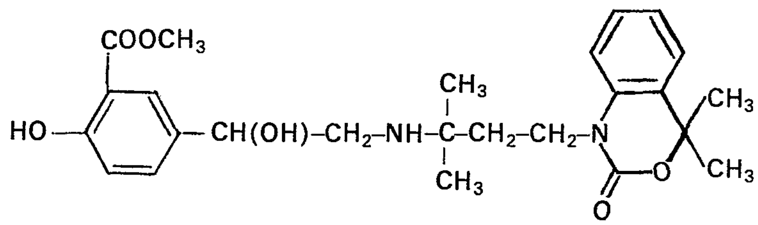

Ein Gemisch von 81,4 g 3-Methoxycarbonyl-4-hydroxy-phenylglyoxal und 79 g 1,1-Dimethyl- 3-[1-(4,4-dimethyl- 3,1-benzoxazin-2-onyl)]-propylamin wird in 1 Ltr. Methanol auf 50 °C erwärmt, dann 3 Stunden bei Raumtemperatur gehalten und anschliessend bei −10 °C unter Rühren portionsweise mit 36 g Natriumborhydrid versetzt. Die Reaktion wird durch 3-stündiges Rühren bei −5 bis 0 °C beendet. Man säuert die Mischung mit 2 N Salzsäure auf pH 2–3 an, destilliert das Lösungsmittel unter Vakuum ab, versetzt den Rückstand bis pH 9–10 mit konz. Ammoniaklösung und schüttelt dreimal mit Essigester aus. Die vereinigten Essigesterphasen werden über Natriumsulfat getrocknet und eingeengt. Der Rückstand enthält 147 g der Titelverbindung. Zur Herstellung des Maleinates wird der Rückstand in 500 ml Acetonitril gelöst und mit 34,8 g Maleinsäure versetzt.

Das Maleinat zeigt den Fp. 139–140 °C.

Entsprechend erhält man nach Verfahren 2):

| Beispiel | Verbindung | Kenndaten |
|---|---|---|
| 64 | 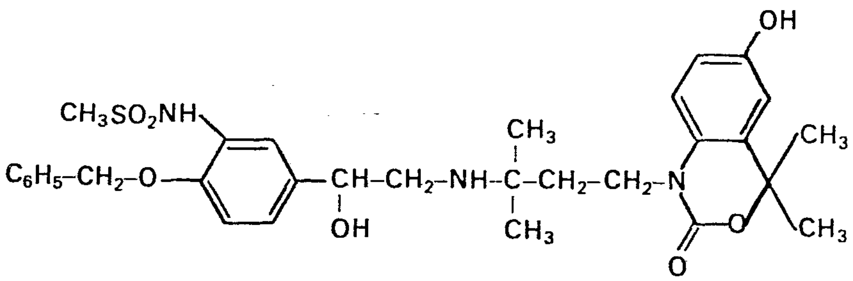 | amorph |
| 65 | 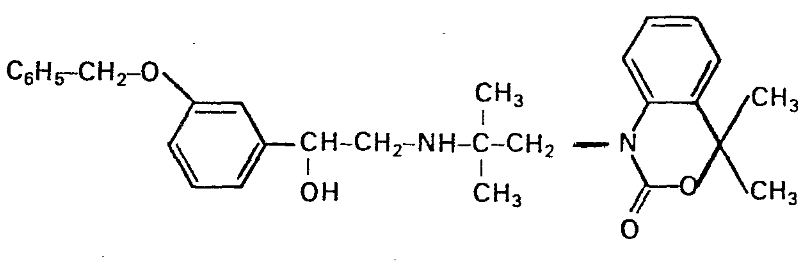 | Fp. Salz: 173 °C Fumarat |

(Fortsetzung)

| Bei-spiel | Verbindung | Kenndaten |
|---|---|---|
| 66 | 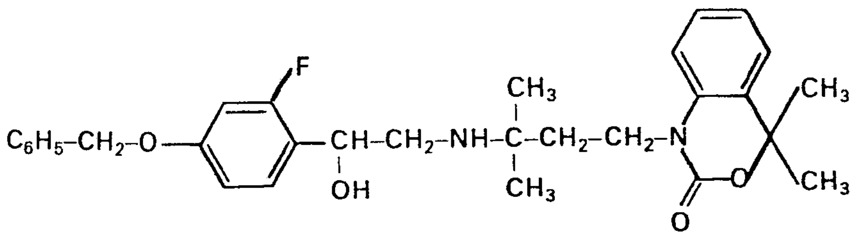 | Fp. Salz: 192 °C Methansulfonat |
| 67 | 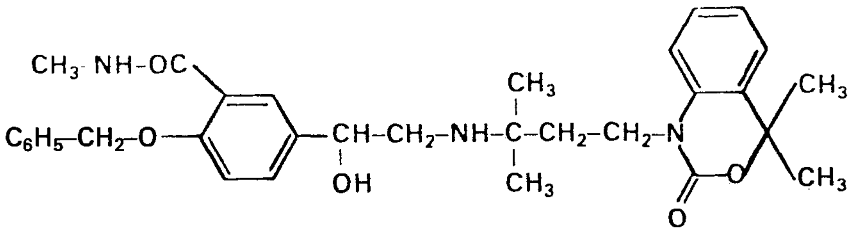 | Fp. Salz: 183 °C Methansulfonat |
| 68 | 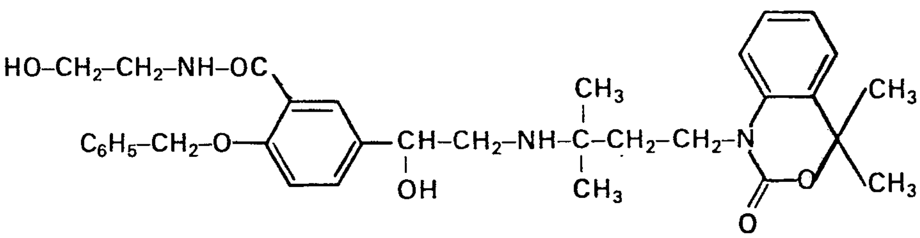 | Fp. Salz: 170 °C Maleinat |
| 69 | 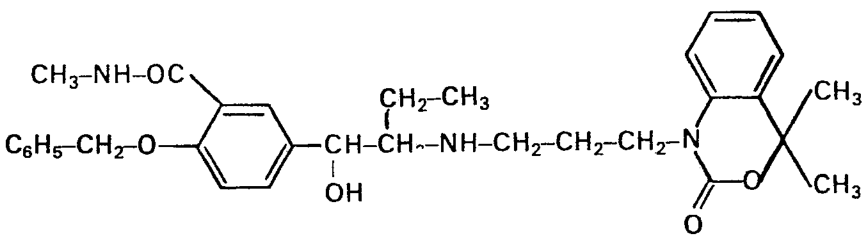 | Fp. Salz: 142 °C Maleinat |
| 70 | 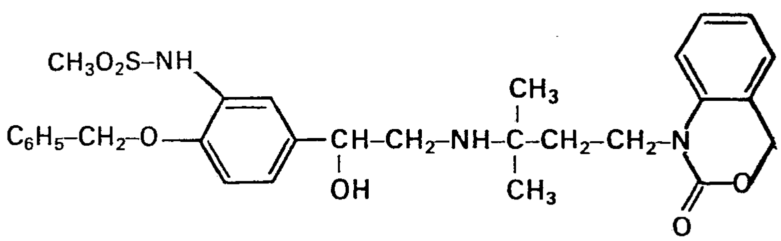 | Fp. Salz: 189 °C Maleinat |
| 71 | 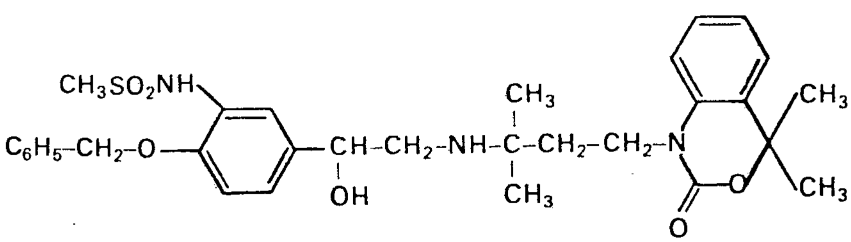 | Fp. Salz: 162 °C Formiat |

(Fortsetzung)

| Beispiel | Verbindung | Kenndaten |
|---|---|---|
| 72 | 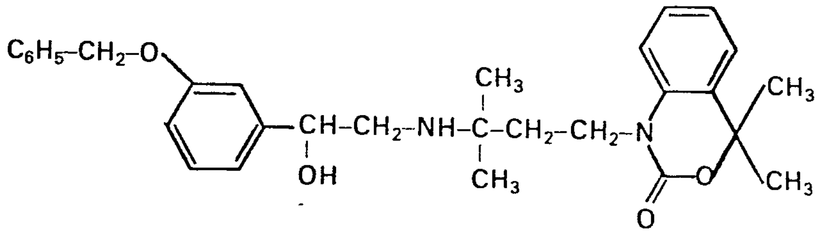 | Fp. Salz: 167 °C Fumarat |
| 73 | 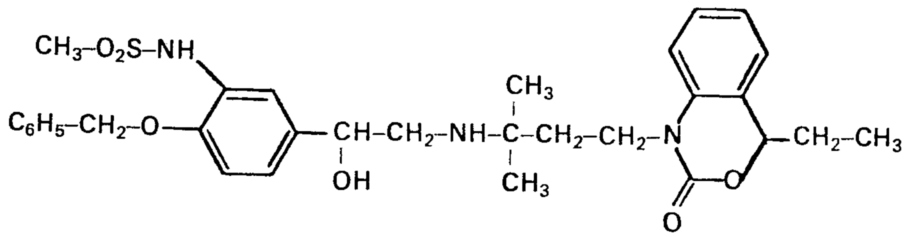 | Fp. Salz: 137 °C Maleinat |
| 74 | 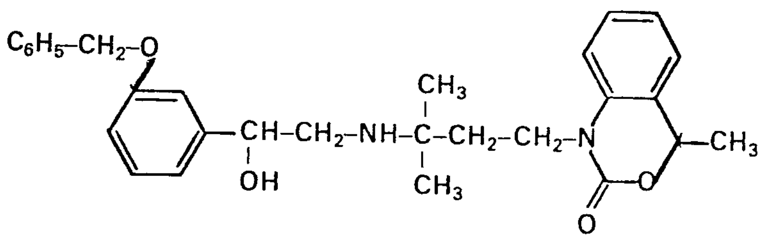 | Fp. Salz: 157 °C Fumarat |
| 75 | 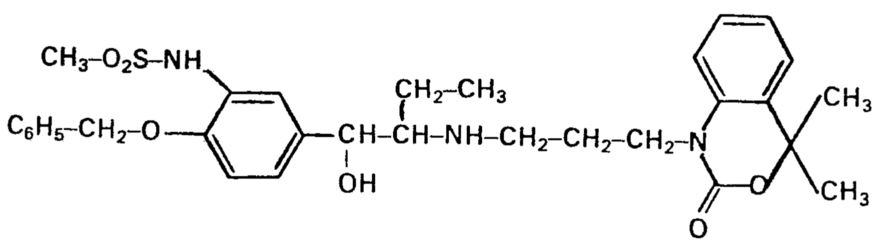 | amorph |
| 76 | 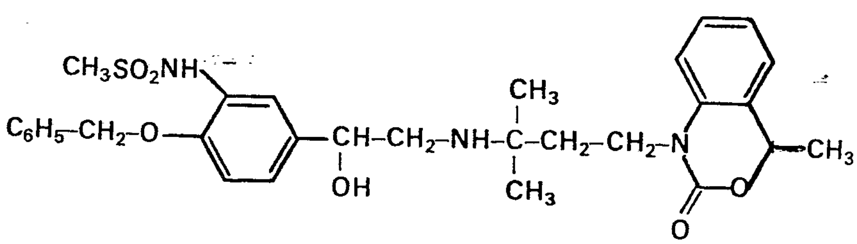 | Fp. Salz: 166 °C Maleinat |
| 77 | 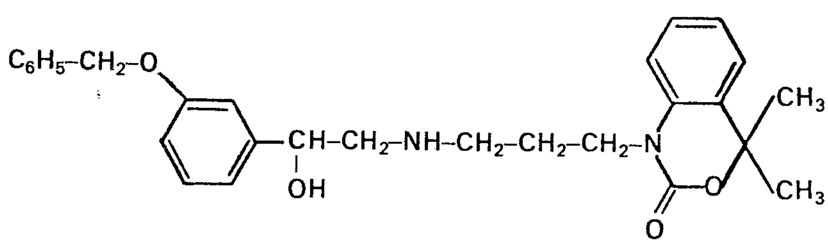 | Fp. Salz: 142 °C Hydrochlorid |

(Fortsetzung)

| Beispiel | Verbindung | Kenndaten |
|---|---|---|
| 78 | 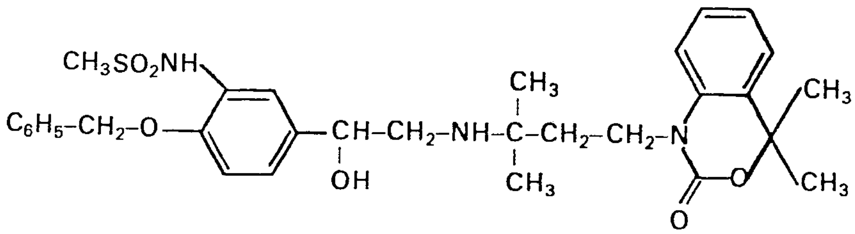 | amorph |
| 79 | 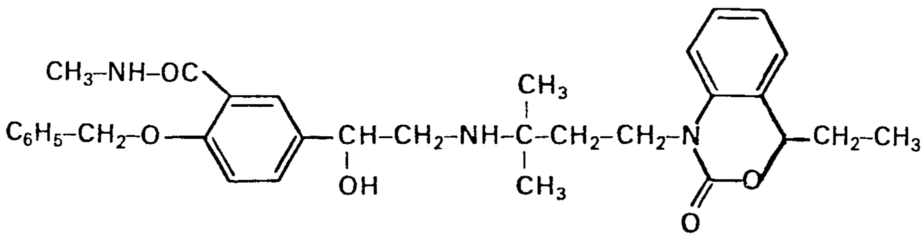 | amorph |
| 80 | 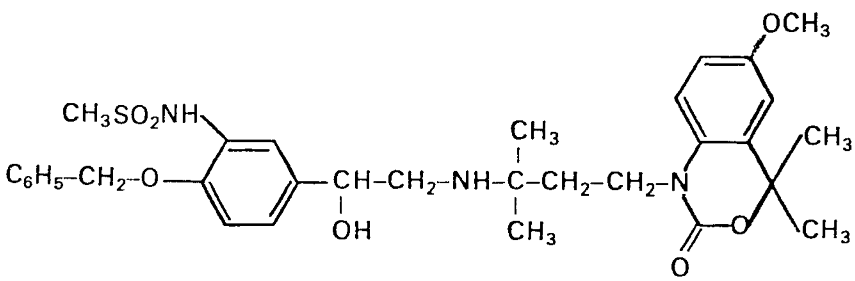 | amorph |
| 81 | 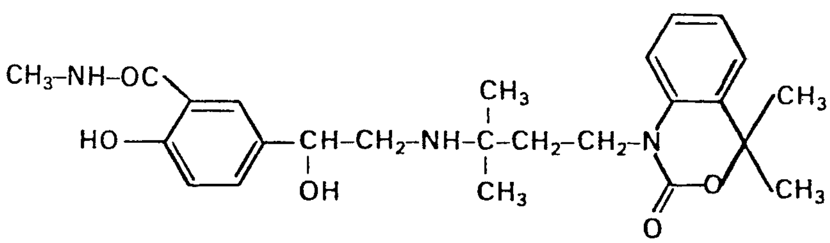 | Fp. Base: 195 °C<br>Fp. Salz: 204 °C<br>Methansulfonat |
| 82 | 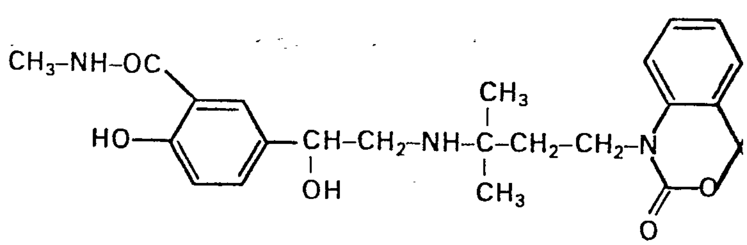 | Fp. Salz: 170 °C<br>Formiat |
| 83 | 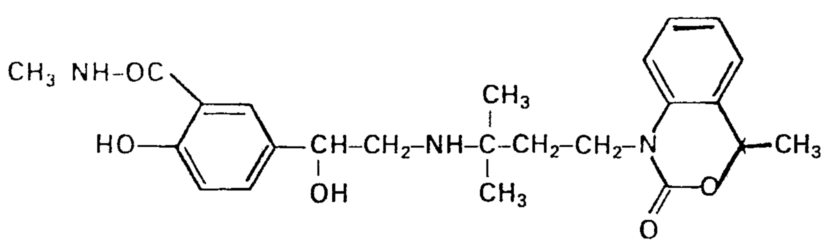 | Fp. Salz: 143 °C<br>Formiat |

(Fortsetzung)

| Beispiel | Verbindung | Kenndaten |
|---|---|---|
| 84 | 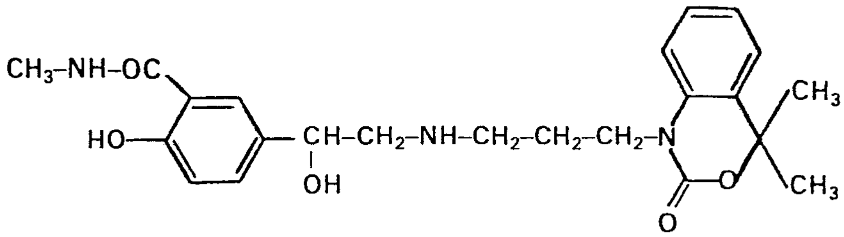 | Fp. Salz: 169 °C<br>Formiat |
| 85 | 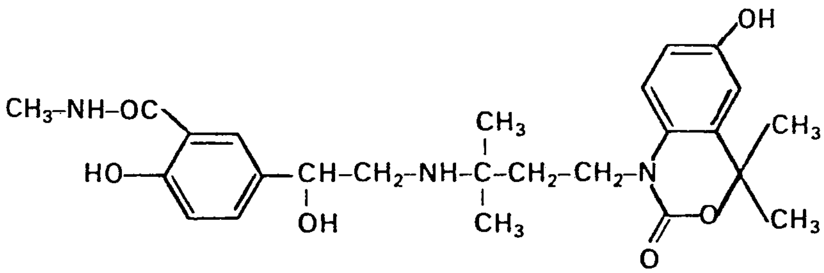 | Fp. Base: 176 °C<br>Fp. Salz: 181 °C<br>Formiat |
| 86 | 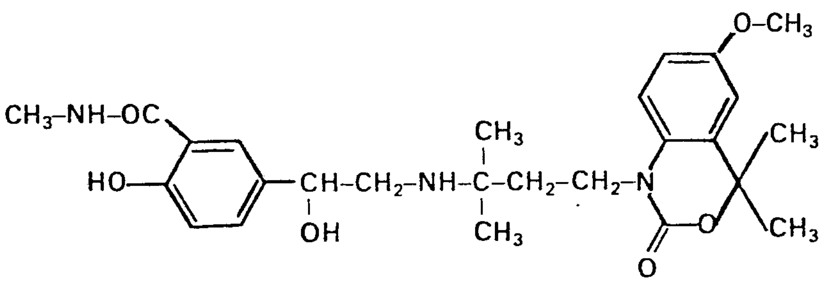 | Fp. Salz: 210 °C<br>Hydrochlorid |
| 87 | 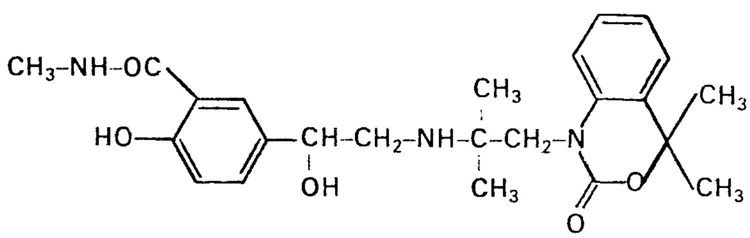 | Fp. Salz: 165 °C<br>p-Aminobenzoat |
| 88 | 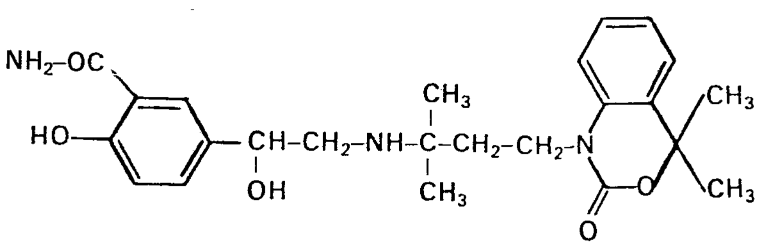 | Fp. Base: 197 °C<br>Fp. Salz: 138 °C<br>Methansulfonat |
| 89 | 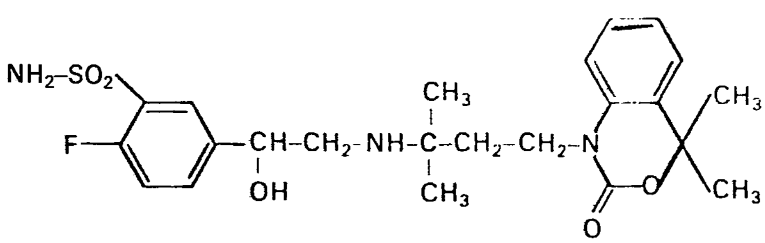 | Fp. Salz: 235 °C<br>Fumarat |

(Fortsetzung)

| Beispiel | Verbindung | Kenndaten |
|---|---|---|
| 90 | 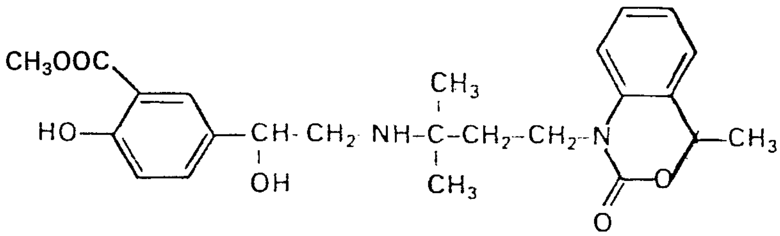 | Fp. Salz: 155 °C p-Aminobenzoat |
| 91 | 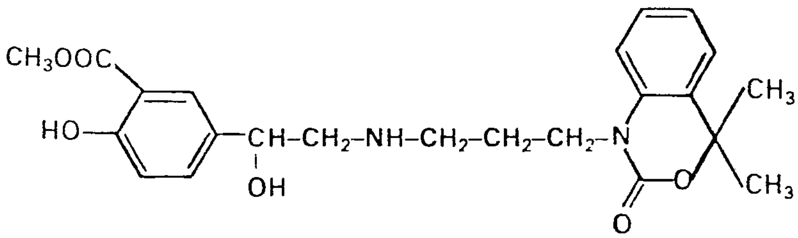 | Fp. Salz: 191 °C Hydrochlorid |
| 92 | 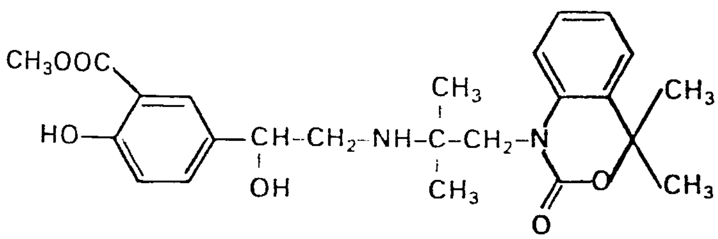 | Fp. Salz: 153 °C p-Aminobenzoat |
| 93 | 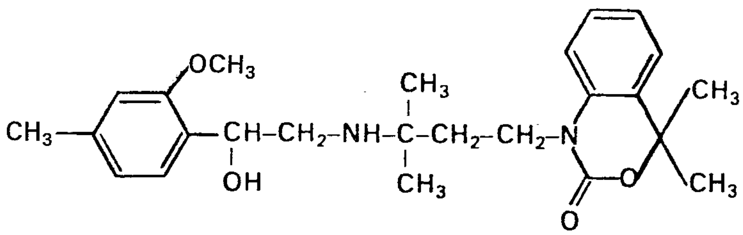 | Fp. Salz: 223 °C Hydrochlorid |
| 94 | 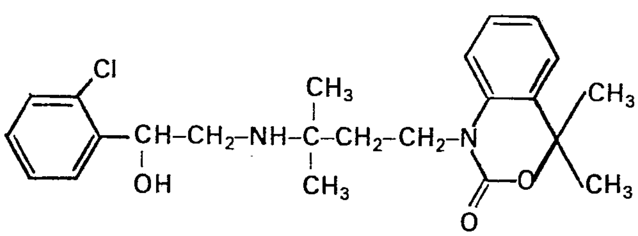 | Fp. Salz: 251 °C Hydrochlorid |
| 95 | 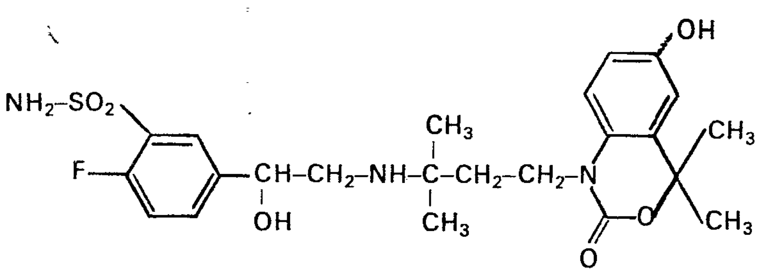 | Fp. Salz: 192 °C Fumarat |

(Fortsetzung)

| Beispiel | Verbindung | Kenndaten |
|---|---|---|
| 96 | 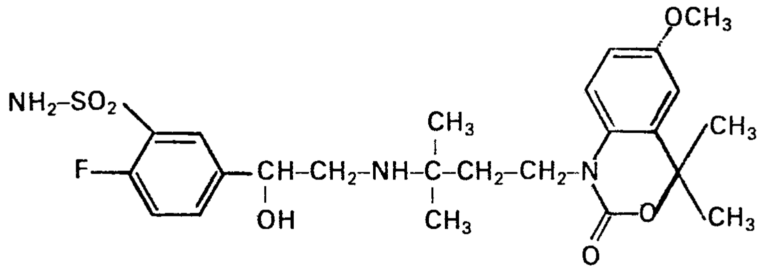 | Fp. Salz: 197 °C Fumarat |
| 97 | 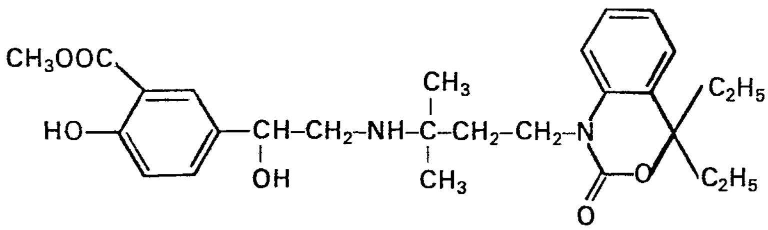 | Fp. Salz: 89 °C Maleinat |
| 98 | 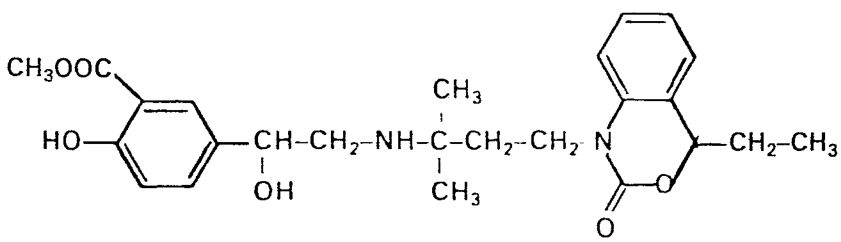 | Fp. Salz: 120 °C Maleinat |
| 99 | 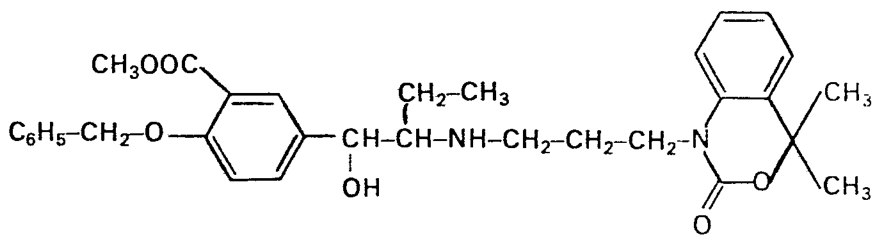 | Fp. Salz: 142 °C Maleinat |
| 100 | 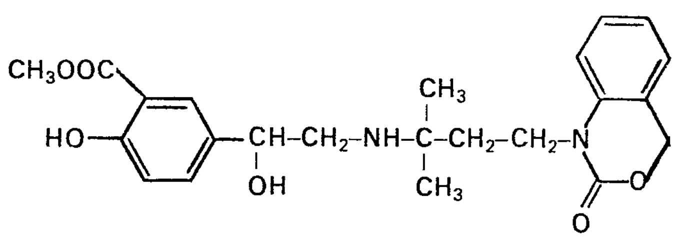 | Fp. Salz: 188 °C p-Aminobenzoat |
| 101 | 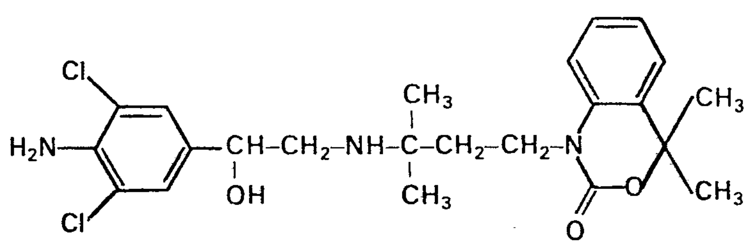 | Fp. Salz: 170 °C Maleinat |

(Fortsetzung)

| Beispiel | Verbindung | Kenndaten |
|---|---|---|
| 102 | 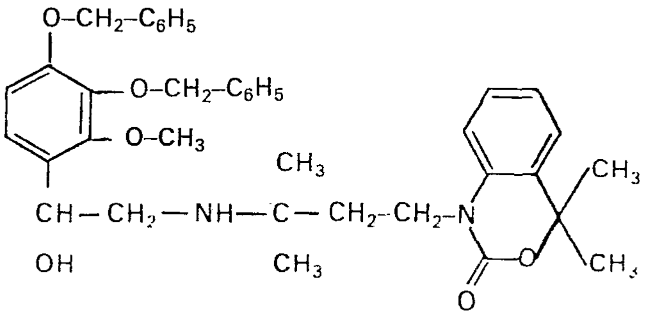 | Fp. Salz: 212 °C Hydrochlorid |
| 103 | 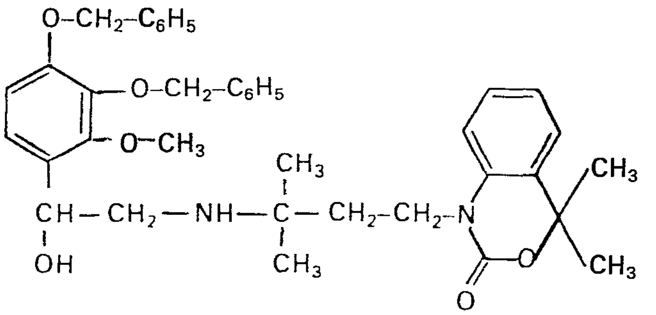 | Fp. Salz: 195 °C Hydrochlorid |

Beispiele für das Verfahren 3

Beispiel 104

1-(3-Methylcarbamoyl-4-hydroxyphenyl)-2-{1,1-dimethyl-3-[1-(4,4-dimethyl-3,1-benzoxazin-2-onyl)]-propylamino}-ethanol

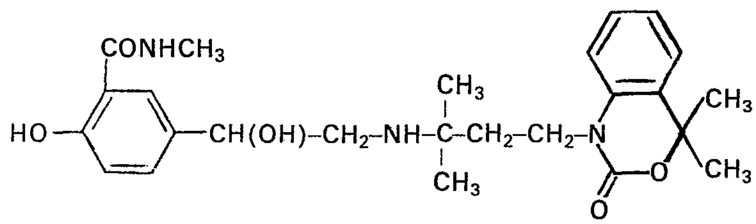

Eine Lösung von 5 g 1-(3-Methylcarbamoyl-4-benzyloxyphenyl)-2-{1,1-dimethyl-3-[1-(4,4-dimethyl-3,1-benzoxazin-2-onyl)]-propylamino}ethanol in 50 ml Methanol wird mit ca. 2,2 ml 13%iger methanolischer Salzsäure auf pH 6,8 eingestellt und in Gegenwart von Pd/C als Katalysator bei Raumtemperatur und Normaldruck hydriert. Nach dem Absaugen des Katalysators wird durch Zugabe von methanolischer Methylaminlösung die Titelverbindung als Base ausgeschieden (Ausbeute 94,7% d.Th., Fp. 195 °C).

Beispiel 105

1-(3-Hydroxyphenyl)-2-{1,1-dimethyl-3-[1-(4,4-dimethyl-3,1-benzoxazin-2-onyl)]-propylamino}-ethanol

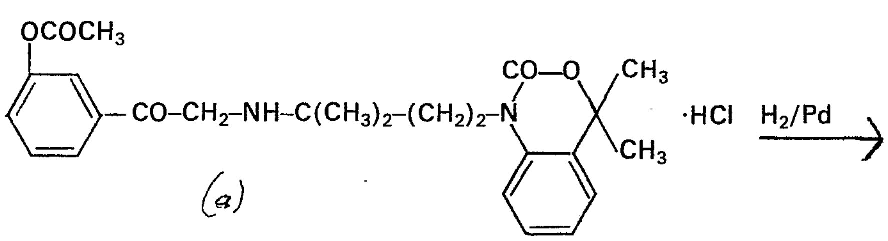

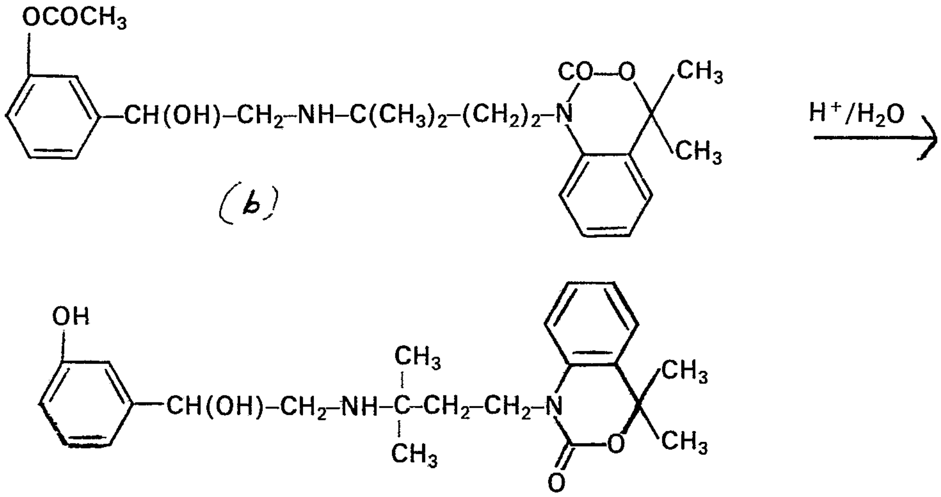


Aus dem Aminoketon-hydrochlorid (Fp. 195–200 °C) der Formel (a) wird mit wässrigem Ammoniak die Base freigesetzt und isoliert. Zur Hydrierung werden 0,7 g der Base in Gegenwart von 0,07 g Platinoxid in 50 ml Methanol bei Raumtemperatur und Normaldruck zum entsprechenden Aminoalkohol (b, Fp. Formiat 102 °C) eingesetzt. Nach dem Abdestillieren des Methanols wird der Rückstand mit 2 ml Methanol und 2 ml 1 N Salzsäure 30 Minuten auf dem Wasserbad gekocht. Die entstandene Titelverbindung wird als Formiat (Fp. 180 °C) in einer Ausbeute von 82% d.Th. isoliert.

Entsprechend erhält man nach dem Verfahren 3:

| Beispiel | Verbindung | Kenndaten |
|---|---|---|
| 106 | 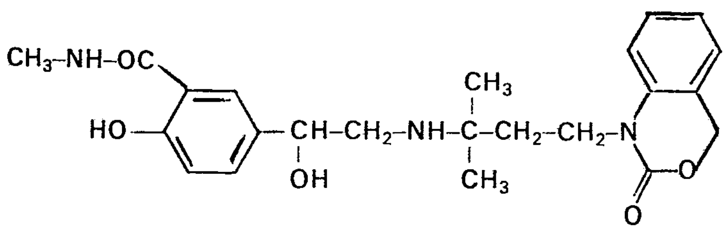 | Fp. Salz: 170 °C Formiat |
| 107 | 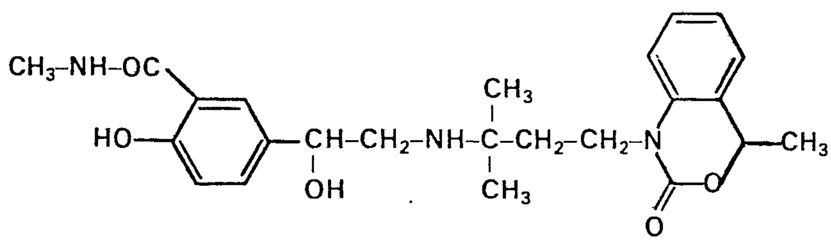 | Fp. Salz: 143 °C Formiat |
| 108 | 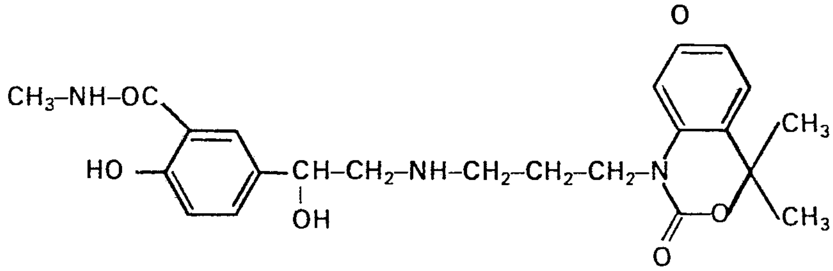 | Fp. Salz: 169 °C Formiat |
| 109 | 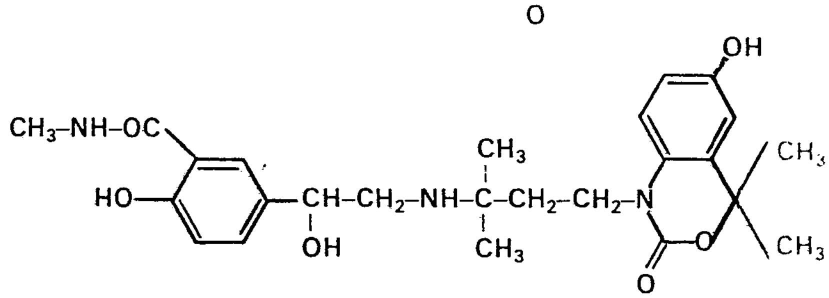 | Fp. Base: 176 °C<br>Fp. Salz: 181 °C Formiat |

(Fortsetzung)

| Beispiel | Verbindung | Kenndaten |
|---|---|---|
| 110 | 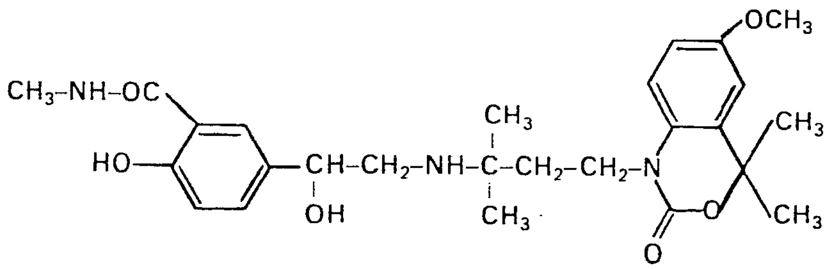 | Fp. Salz: 210 °C<br>Hydrochlorid |
| 111 | 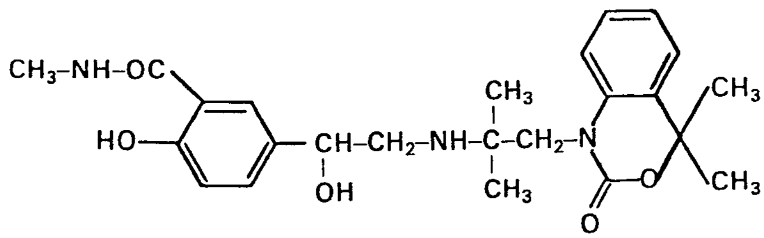 | Fp. Salz: 165 °C<br>p-Aminobenzoat |
| 112 | 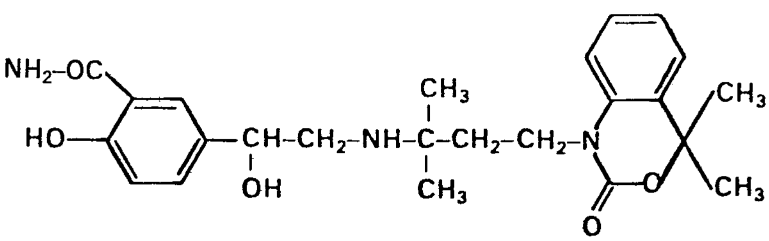 | Fp. Base: 197 °C<br>Fp. Salz: 138 °C<br>Methansulfonat |
| 113 | 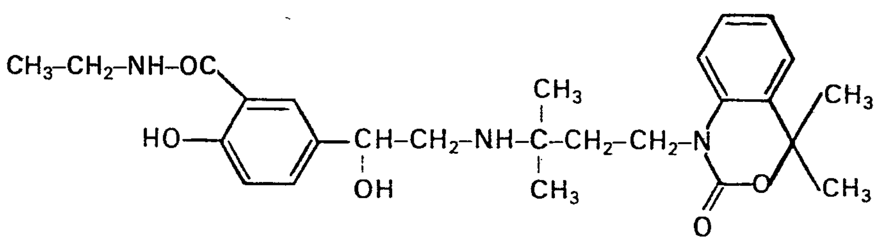 | Fp. Salz: 200 °C<br>Methansulfonat |
| 114 | 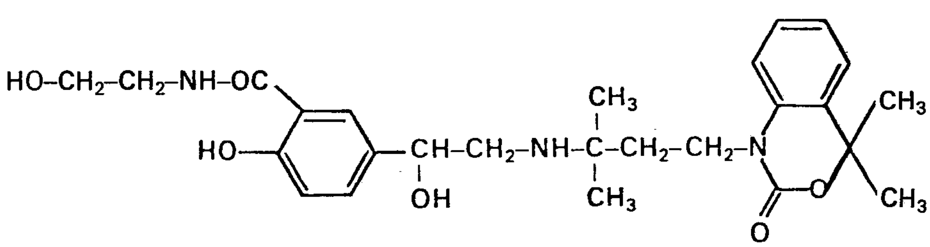 | Fp. Base: 161 °C<br>Fp. Salz: 181 °C<br>Hydrochlorid |
| 115 | 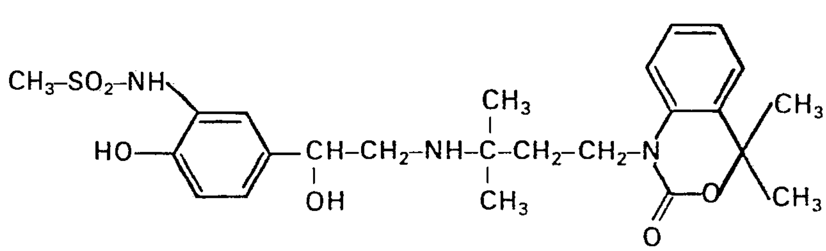 | Fp. Salz: 164 °C<br>Formiat |

(Fortsetzung)

| Beispiel | Verbindung | Kenndaten |
|---|---|---|
| 116 | 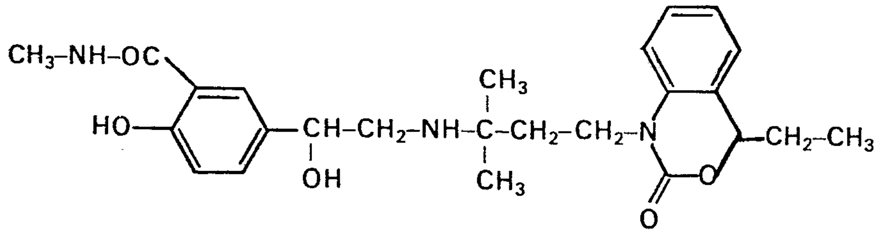 | Fp. Base: 163 °C<br>Fp. Salz: 181 °C<br>Hydrochlorid |
| 117 | 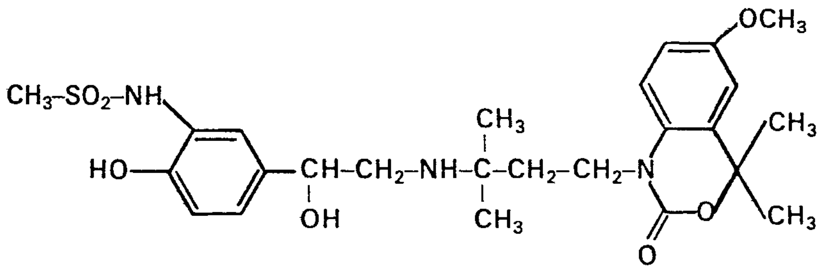 | Fp. Base: 181 °C<br>Fp. Salz: 183 °C<br>Hydrochlorid |
| 118 | 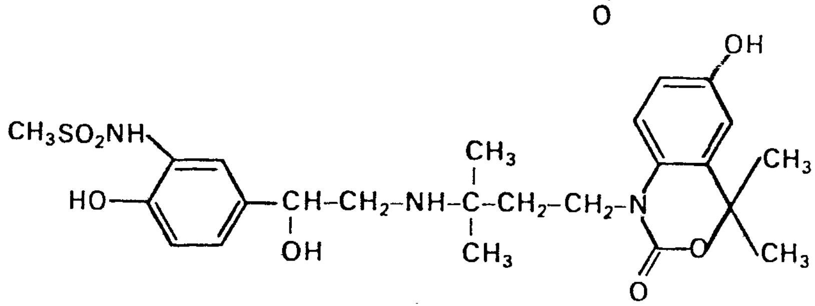 | Fp. Salz: 211 °C<br>Hydrochlorid |
| 119 | 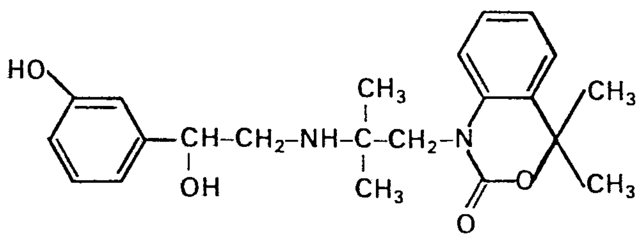 | Fp. Base: 140 °C<br>Fp. Salz: 166 °C<br>Fumarat |
| 120 | 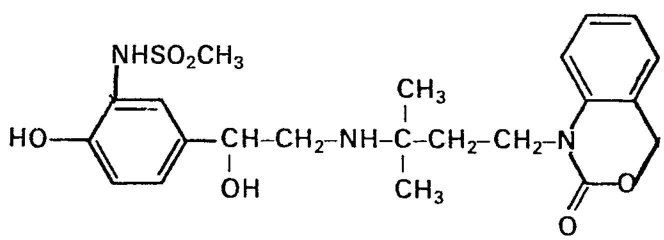 | Fp. Base: 200 °C<br>Fp. Salz: 180 °C<br>Formiat |
| 121 | 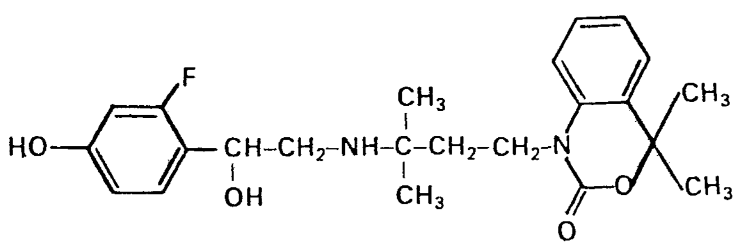 | Fp. Salz: 194 °C<br>Methansulfonat |

(Fortsetzung)

| Beispiel | Verbindung | Kenndaten |
|---|---|---|
| 122 | 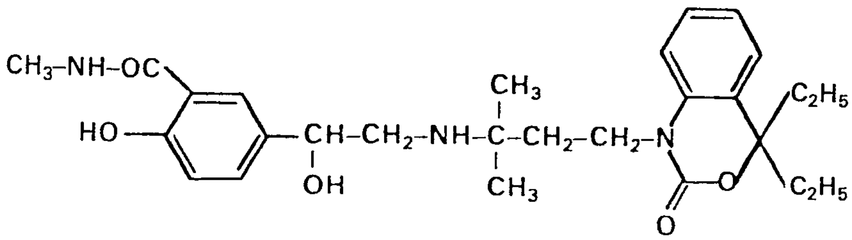 | Fp. Salz: 214 °C Methansulfonat |
| 123 | 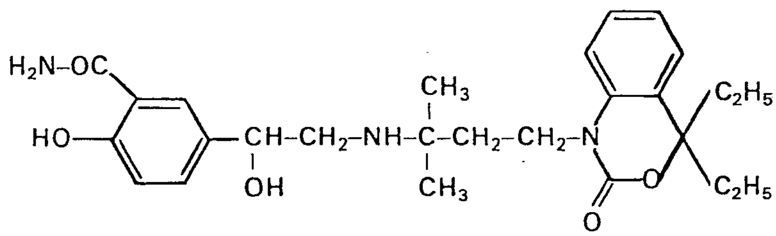 | Fp. Base: 183 °C<br>Fp. Salz: 208 °C Hydrochlorid |
| 124 | 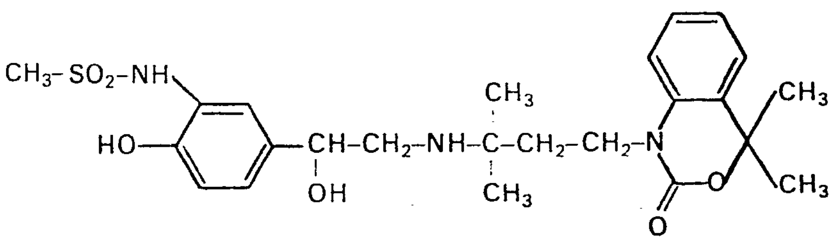 | Fp. Base: 192 °C |
| 125 | 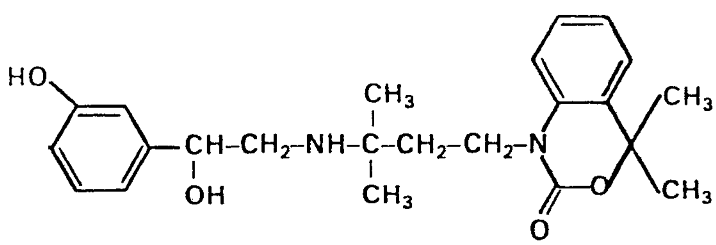 | Fp. Salz: 180 °C Formiat |
| 126 | 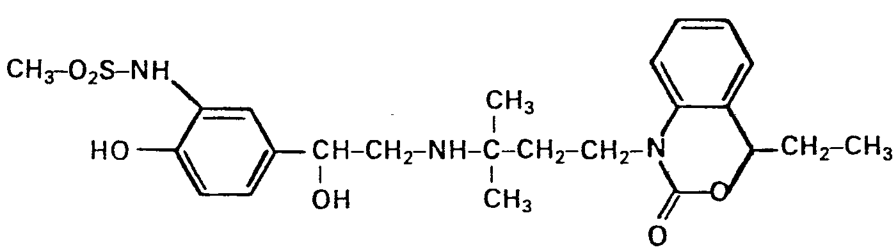 | Fp. Salz: 182 °C Hydrochlorid |
| 127 | 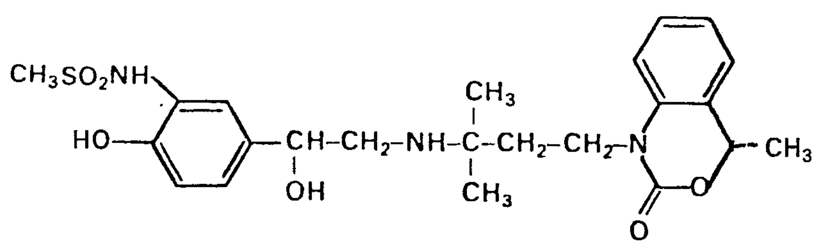 | Fp. Salz: 192 °C Formiat |

(Fortsetzung)

| Beispiel | Verbindung | Kenndaten |
|---|---|---|
| 128 | 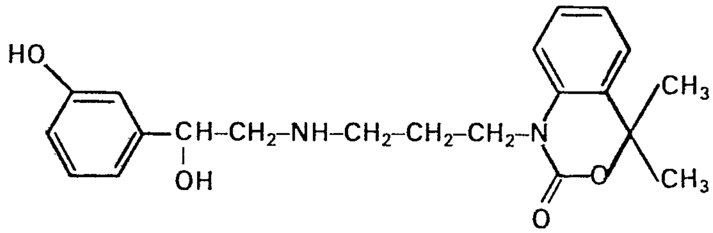 | Fp. Salz: 147 °C Formiat |
| 129 | 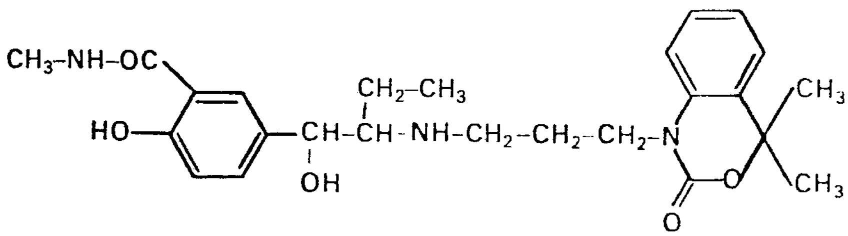 | Fp. Salz: 165 °C Methansulfonat |
| 130 | 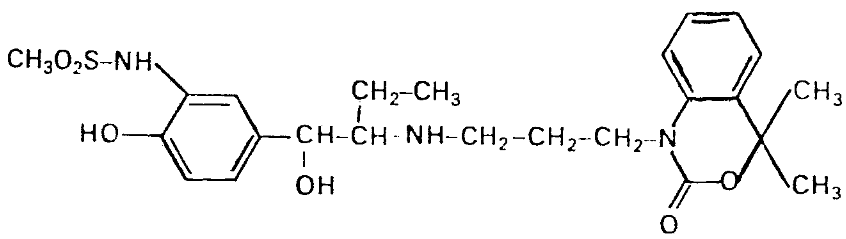 | Fp. Salz: 183 °C Succinat |
| 131 | 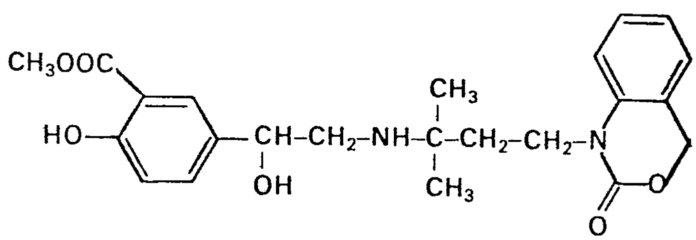 | Fp. Salz: 188 °C p-Aminobenzoat |
| 132 | 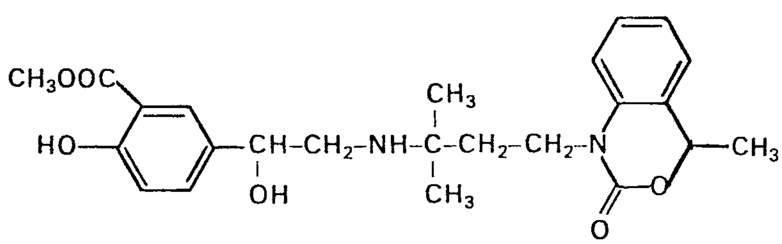 | Fp. Salz: 155 °C p-Aminobenzoat |
| 133 | 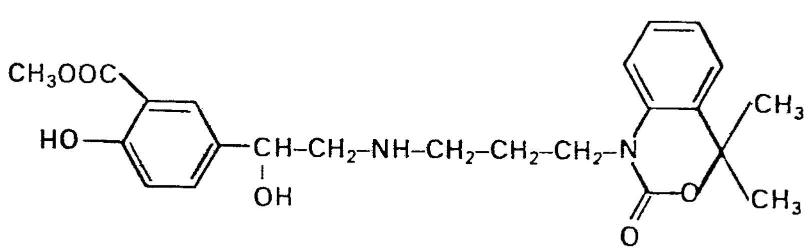 | Fp. Salz: 191 °C Hydrochlorid |

(Fortsetzung)

| Beispiel | Verbindung | Kenndaten |
|---|---|---|
| 134 | 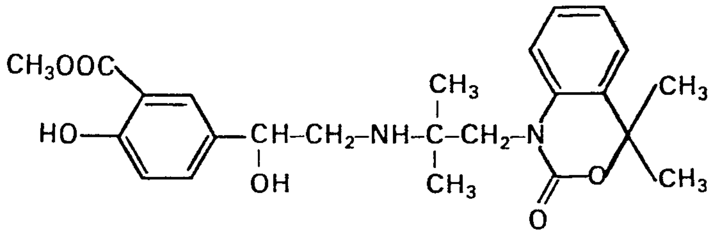 | Fp. Salz: 153 °C p-Aminobenzoat |
| 135 | 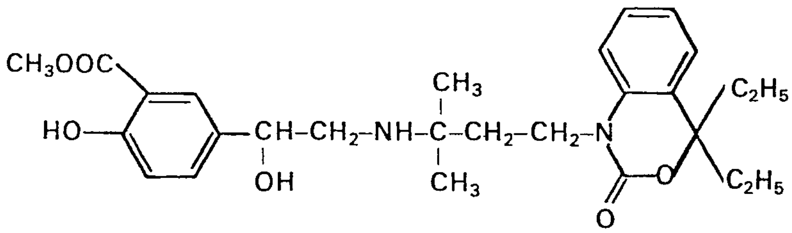 | Fp. Salz: 89 °C Maleinat |
| 136 | 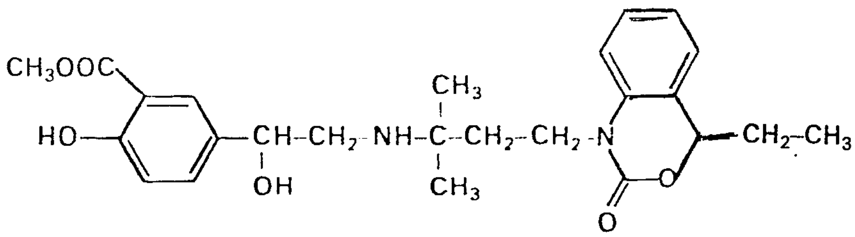 | Fp. Salz: 120 °C Maleinat |
| 137 | 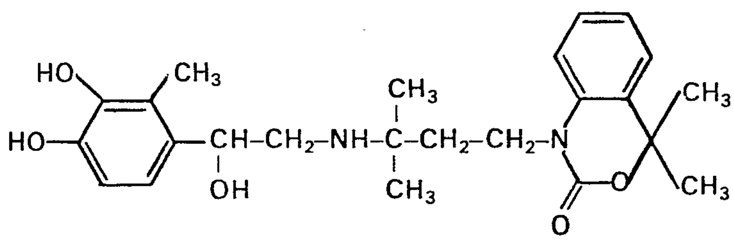 | Fp. Salz: 183 °C Formiat |
| 138 | 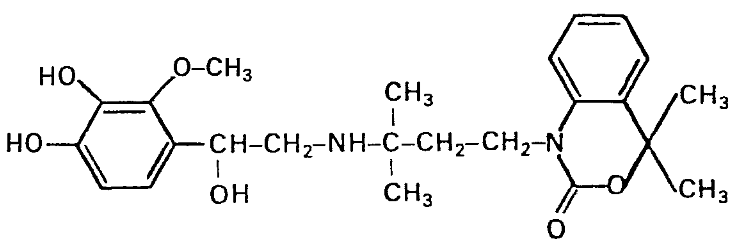 | Fp. Salz: 172 °C Formiat |

Beispiele für das Verfahren 4

Beispiel 139

1-(3-Methylcarbamoyl-4-hydroxyphenyl)-2-{1,1-dimethyl-3-[1-(4,4-dimethyl-3,1-benzoxazin-2-onyl)]-propylamino}-ethanol

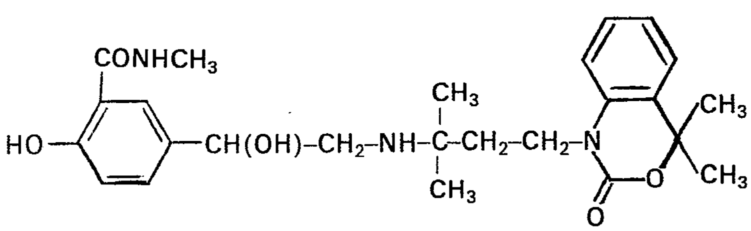

Eine Mischung von 9,1 g 1-(3-Methoxycarbonyl-4-hydroxyphenyl)- 2-{1,1-dimethyl-3-[1-(4,4-dimethyl- 3,1-benzoxazin-2-onyl)]-propylamino}-ethanol und 10 ml Methylamin in 50 ml Methanol setzt man durch 4-tägiges Stehen bei Raumtemperatur zu der Titelverbindung um.

Man isoliert sie nach dem Abdestillieren des Lösungsmittels als Formiat in Acetonitril als Lösungsmittel (Fp. 174 °C, Ausbeute 9,7 g).

Mit wässriger Ammoniaklösung wird aus dem Formiat die Base (Fp. 194 °C) erhalten.

Entsprechend erhält man nach dem Verfahren 4:

| Beispiel | Verbindung | Kenndaten |
|---|---|---|
| 140 | 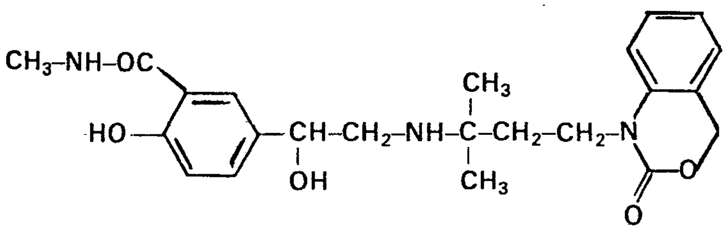 | Fp. Salz: 170 °C Formiat |
| 141 | 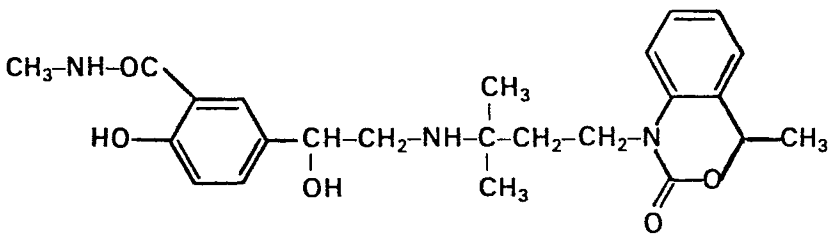 | Fp. Salz: 143 °C Formiat |
| 142 | 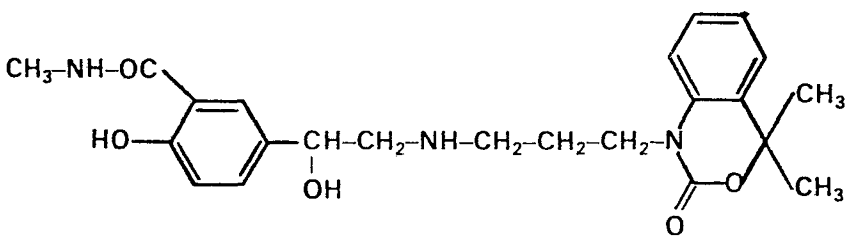 | Fp. Salz: 169 °C Formiat |
| 143 | 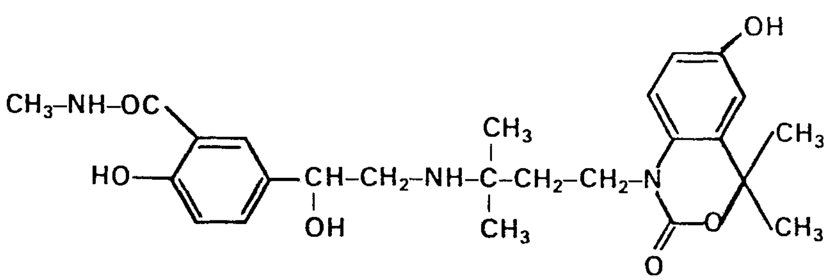 | Fp. Base: 176 °C Fp. Salz: 181 °C Formiat |
| 144 | 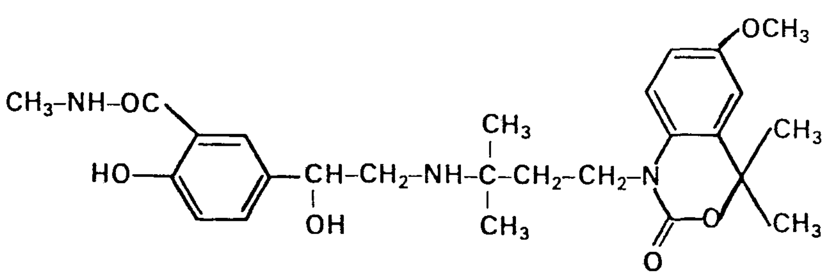 | Fp. Salz: 210 °C Hydrochlorid |

(Fortsetzung)

| Bei-spiel | Verbindung | Kenndaten |
|---|---|---|
| 145 | 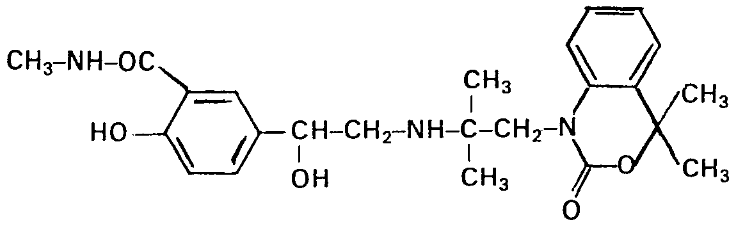 | Fp. Salz: 165 °C<br>p-Aminobenzoat |
| 146 | 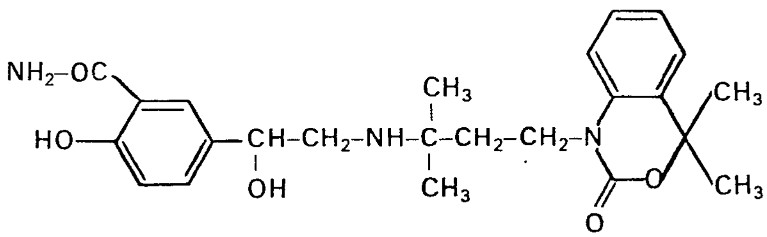 | Fp. Base: 197 °C<br>Fp. Salz: 138 °C<br>Methansulfonat |
| 147 | 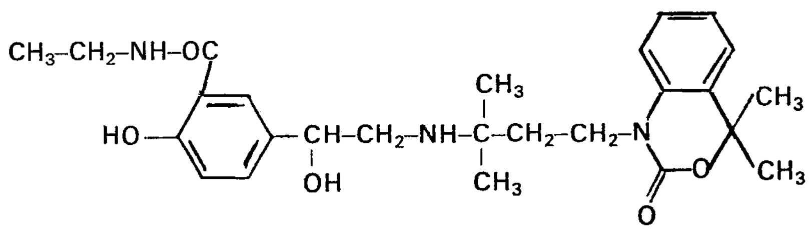 | Fp. Salz: 200 °C<br>Methansulfonat |
| 148 | 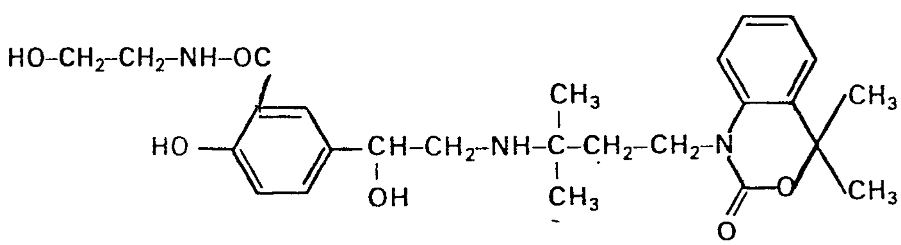 | Fp. Base: 161 °C<br>Fp. Salz: 181 °C<br>Hydrochlorid |
| 149 | 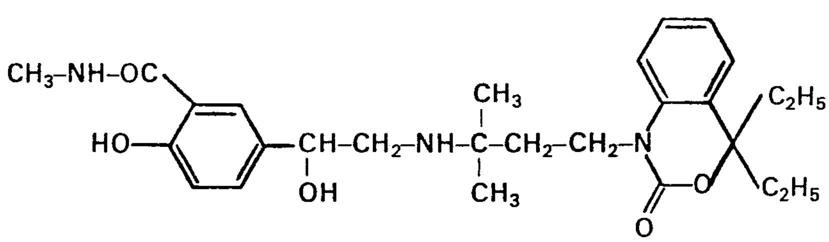 | Fp. Salz: 214 °C<br>Methansulfonat |
| 150 | 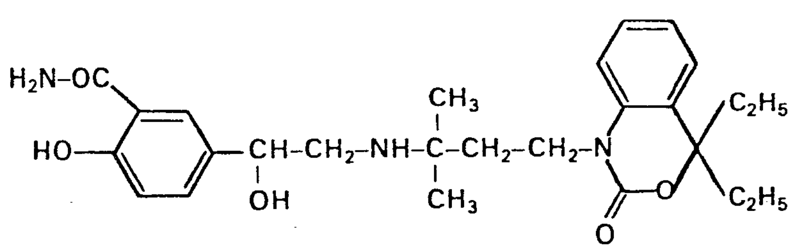 | Fp. Base: 183 °C<br>Fp. Salz: 208 °C<br>Hydrochlorid |

(Fortsetzung)

| Beispiel | Verbindung | Kenndaten |
|---|---|---|
| 151 | 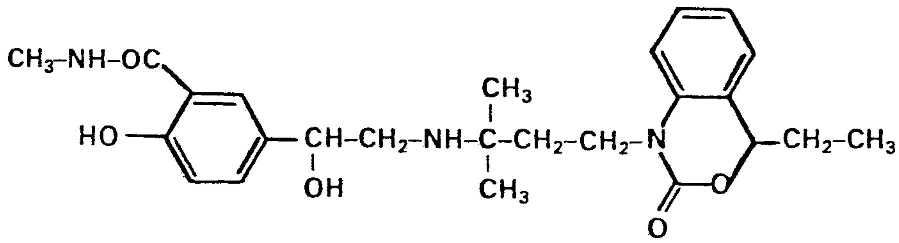 | Fp. Base: 163 °C<br>Fp. Salz: 181 °C<br>Hydrochlorid |
| 152 | 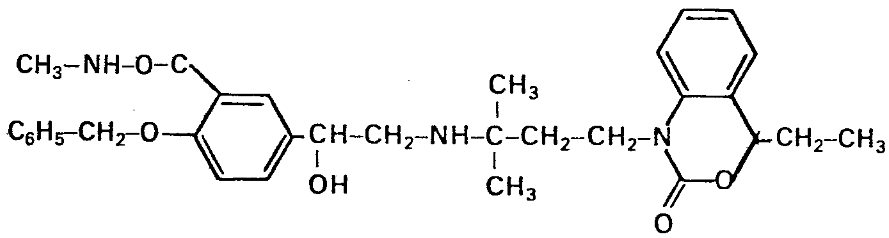 | amorph |
| 153 | 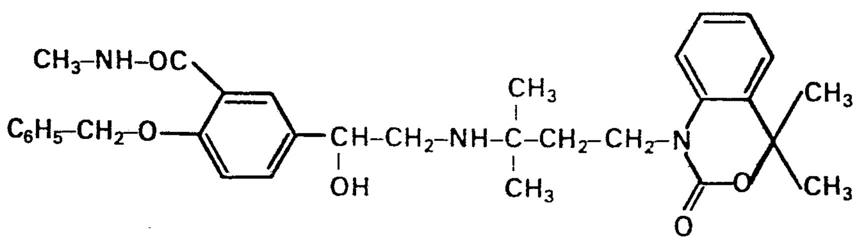 | Fp. Salz: 183 °C<br>Methansulfonat |
| 154 | 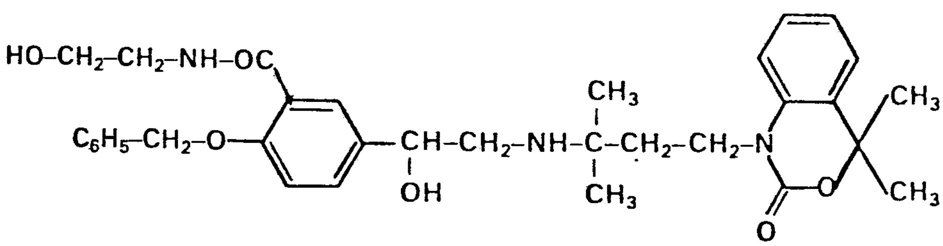 | Fp. Salz: 170 °C<br>Maleinat |
| 155 | 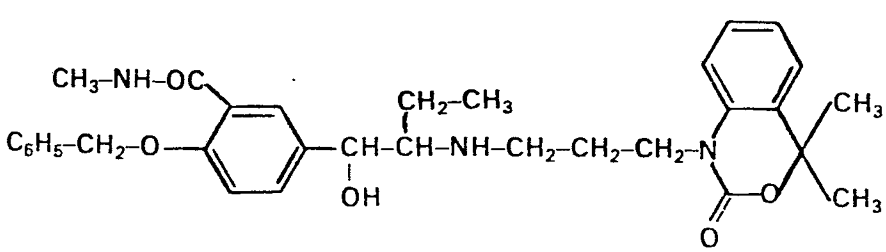 | Fp. Salz: 142 °C<br>Maleinat |

**Patentansprüche**

1. Verbindungen der allgemeinen Formel

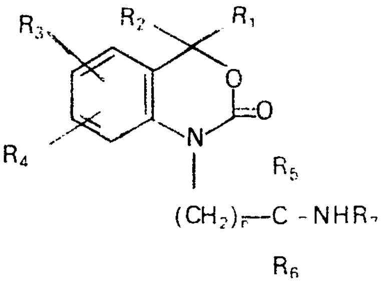

(I),

in der

$R_1$ und $R_2$, die gleich oder verschieden sein können, Wasserstoff oder $(C_1–C_4)$-Alkyl

$R_3$ und $R_4$, die gleich oder verschieden sein können, Wasserstoff, Fluor, Chlor, Hydroxy, Methyl, Ethyl, $(C_1–C_4)$-Alkoxy oder auch gemeinsam Methylendioxy,

$R_5$ und $R_6$, die gleich oder verschieden sein können, Wasserstoff oder Methyl,

$R_7$ die Gruppe

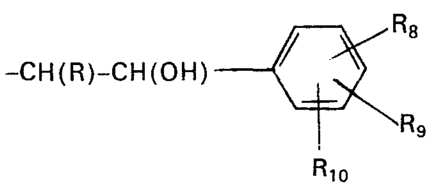

(II),

R Wasserstoff oder $(C_1–C_4)$-Alkyl,

$R_8$ Fluor, Chlor, $(C_1–C_4)$-Alkyl, $(C_1–C_4)$-Alkylthio, Hydroxymethyl oder eine der Gruppen $CONHR_{11}$, $SO_2NHR_{11}$, $OR_{12}$, Methoxycarbonyl, Ethoxycarbonyl oder $NHSO_2CH_3$,

$R_9$ Wasserstoff, Fluor, Chlor oder $OR_{12}$

$R_{10}$ Wasserstoff, Amino, Methyl, Methoxy oder Chlor,

$R_{11}$ Wasserstoff, Methyl, Ethyl oder Hydroxyethyl,

$R_{12}$ Wasserstoff, $(C_1–C_4)$-Alkyl, $(C_1–C_4)$-Alkyl-CO, $C_6H_5–CH_2$ oder $C_6H_5–CO$ und

n 1, 2 oder 3 bedeutet, in Form von Racematen, gegebenenfalls diastereomeren Antipodenpaaren sowie einzelnen Enantiomerer, jeweils als freie Basen oder als Säureadditionssalze.

2. Verbindungen nach Anspruch 1, dadurch gekennzeichnet, dass

$R_1/R_2$ $CH_3/CH_3$ oder $CH_3/H$,

$R_3$ H, OH oder $OCH_3$,

$R_4$ H,

$R_5/R_6$ $CH_3/CH_3$, $CH_3/H$ oder H/H,

$R_7$ 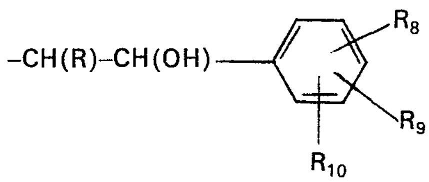

R H, $CH_3$ oder $C_2H_5$

$R_8$ $CONHR_{11}$, $SO_2NHR_{11}$, $NHSO_2CH_3$, $OR_{12}$, F, Cl, COOAlkyl oder $CH_2OH$,

$R_9$ $OR_{12}$, F, Cl

$R_{10}$ H, $NH_2$, $CH_3$, $OCH_3$ oder Cl

$R_{11}$ H, $CH_3$ oder $C_2H_5$

$R_{12}$ H, $C_6H_5–CH_2$ oder $C_6H_5–CO$ und

n 1 oder 2 bedeutet.

3. Arzneimittel, gekennzeichnet durch einen Gehalt an einer Verbindung nach Anspruch 1 oder 2 in Verbindung mit üblichen Hilfs- und/oder Trägerstoffen.

4. Verfahren zur Herstellung von Verbindungen der allgemeinen Formel

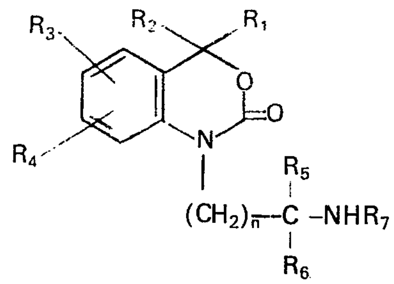

(I),

in der

$R_1$ und $R_2$, die gleich oder verschieden sein können, Wasserstoff oder $(C_1–C_4)$-Alkyl

$R_3$ und $R_4$, die gleich oder verschieden sein können, Wasserstoff, Fluor, Chlor, Hydroxy, Methyl, Ethyl, $(C_1–C_4)$-Alkoxy oder auch gemeinsam Methylendioxy,

$R_5$ und $R_6$, die gleich oder verschieden sein können, Wasserstoff oder Methyl,

$R_7$ die Gruppe

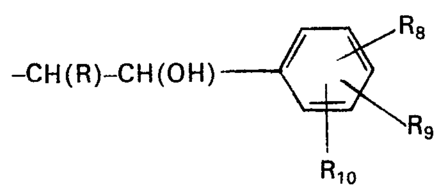

(II),

R Wasserstoff oder $(C_1–C_4)$-Alkyl,

$R_8$ Fluor, Chlor, $(C_1–C_4)$-Alkyl, $(C_1–C_4)$-Alkylthio, Hydroxymethyl oder eine der Gruppen $CONHR_{11}$, $SO_2NHR_{11}$, $OR_{12}$, Methoxycarbonyl, Ethoxycarbonyl oder $NHSO_2CH_3$,

$R_9$ Wasserstoff, Fluor, Chlor oder $OR_{12}$

$R_{10}$ Wasserstoff, Amino, Methyl, Methoxy oder Chlor,

$R_{11}$ Wasserstoff, Methyl, Ethyl oder Hydroxyethyl,

$R_{12}$ Wasserstoff, $(C_1–C_4)$-Alkyl, $(C_1–C_4)$-Alkyl-CO, $C_6H_5–CH_2$ oder $C_6H_5–CO$ und

n 1, 2 oder 3 bedeutet, in Form von Racematen, gegebenenfalls diastereomeren Antipodenpaaren sowie einzelnen Enantiomerer, jeweils als freie Basen oder als Säureadditionssalze, dadurch gekennzeichnet, dass man

a) eine Verbindung der allgemeinen Formel

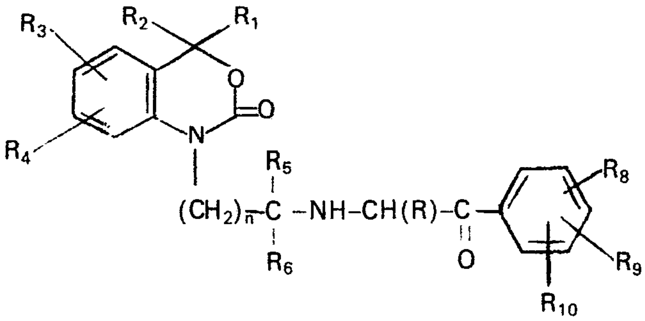

(II)

worin R, $R_1$–$R_{10}$ und n die obige Bedeutung haben, reduziert oder dass man

b) eine Verbindung der allgemeinen Formel

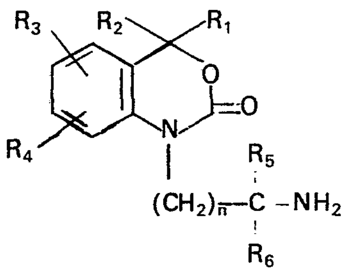

(III),

worin n und $R_2$–$R_6$ die obige Bedeutung haben, mit einer Verbindung der allgemeinen Formel

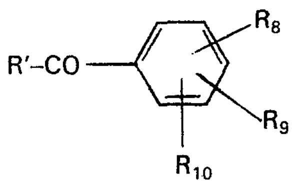

(IV),

worin $R_8$, $R_9$ und $R_{10}$ die obige Bedeutung haben und R' für eine der Gruppen

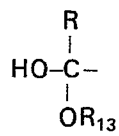 oder 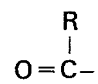

(Va) (Vb)

steht, worin R die obige Bedeutung hat und $R_{13}$ $C_1$–$C_4$-Alkyl ist, reduktiv alkyliert oder dass man

c) aus einer Verbindung der allgemeinen Formel

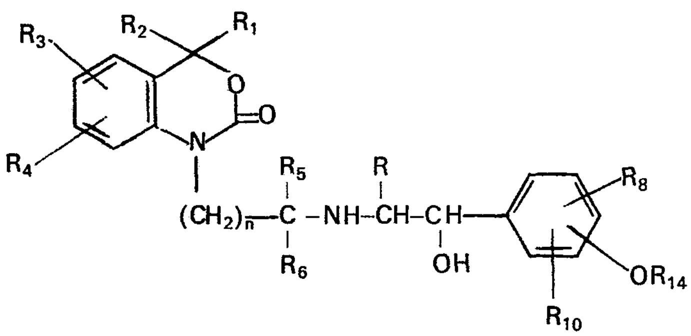

(VII)

worin n, R und $R_1$–$R_{10}$ die obige Bedeutung haben und $R_{14}$ für Benzyl, CO–($C_1$–$C_4$-Alkyl) oder CO–$C_6H_5$ steht, die Schutzgruppe $R_{14}$ abspaltet oder dass man

d) eine Verbindung der allgemeinen Formel

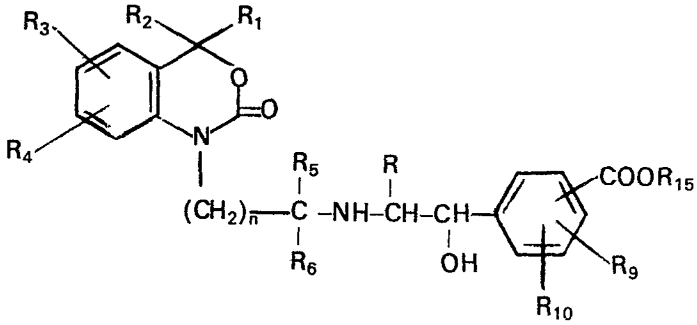

(IX),

worin n, R und $R_1$–$R_{10}$ die obige Bedeutung haben und $R_{15}$ für einen ($C_1$–$C_4$)-Alkyl- oder Aralkylrest steht, mit einem Amin der allgemeinen Formel

$H_2NR_{11}$ (X),

worin $R_{11}$ die obige Bedeutung hat, umsetzt und dass man gewünschtenfalls nach den Verfahren a) bis d) erhaltene Produkte in diastereomere Antipodenpaare oder in die einzelnen Enantiomeren auftrennt und/oder primär erhaltene Basen in Säureadditionssalze, primär erhaltene Säureadditionssalze in freie Basen umwandelt.

**Revendications**

1. Composés de formule générale:

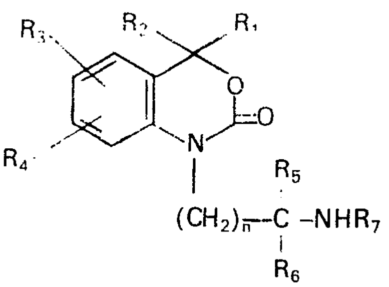

(I),

dans laquelle:

$R_1$ et $R_2$, qui peuvent être identiques ou différents, représentent hydrogène ou alcoyle en $C_1$–$C_4$,

$R_3$ et $R_4$, qui peuvent être identiques ou différents, représentent hydrogène, fluor, chlore, hydroxy, méthyle, éthyle, alcoxy en $C_1$–$C_4$ ou également ensemble méthylènedioxy,

$R_5$ et $R_6$, qui peuvent être identiques ou différents, représentent hydrogène ou méthyle,

$R_7$ représente le groupe

-CH(R)-CH(OH)— 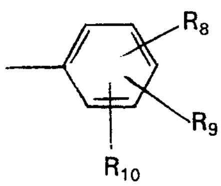 (II),

R représente hydrogène ou alcoyle en $C_1$–$C_4$,

$R_8$ représente fluor, chlore, alcoyle en $C_1$–$C_4$, alcoylthio en $C_1$–$C_4$, hydroxyméthyle ou l'un des groupes $CONHR_1$, $SO_2NHR_{11}$, $OR_{12}$, méthoxycarbonyle, éthoxycarbonyle ou $NHSO_2CH_3$,

$R_9$ représente hydrogène, fluor, chlore ou $OR_{12}$,

$R_{10}$ représente hydrogène, amino, méthyle, méthoxy ou chlore,

$R_{11}$ représente hydrogène, méthyle, éthyle ou hydroxyéthyle

$R_{12}$ représente hydrogène, alcoyle en $C_1$–$C_4$, alcoyle en $C_1$–$C_4$–CO, $C_6H_5CH_2$ ou $C_6H_5$–CO, et

n représente 1, 2 ou 3, sous forme de racémates, éventuellement de paires d'antipodes diastéréoisomères ainsi que d'énantiomères individuels, dans chaque cas sous forme de bases libres ou sous forme de sels d'addition d'acides.

2. Composés selon la revendication 1, caractérisés en ce que:

$R_1/R_2$ représente $CH_3/CH_3$ ou $CH_3/H$,

$R_3$ représente H, OH ou $OCH_3$,

$R_4$ représente H,

$R_5/R_6$ représente $CH_3/CH_3$, $CH_3/H$ ou H/H,

$R_7$ représente –CH(R)–CH(OH)— 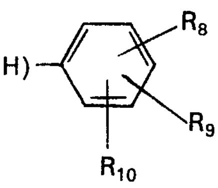

R représente H, $CH_3$ ou $C_2H_5$,

$R_8$ représente $CONHR_{11}$, $SO_2NHR_{11}$, $NHSO_2CH_3$, $OR_{12}$, F, Cl, COO-alcoyle ou $CH_2OH$,

$R_9$ représente $OR_{12}$, F, Cl,

$R_{10}$ représente H, $NH_2$, $CH_3$, $OCH_3$ ou Cl,

$R_{11}$ représente H, $CH_3$ ou $C_2H_5$,

$R_{12}$ représente H, $C_6H_5$–$CH_2$ ou $C_6H_5$–CO et

n représente 1 ou 2.

3. Médicament caractérisé par une teneur en un composé selon la revendication 1 ou 2 en combinaison avec des adjuvants et/ou excipients usuels.

4. Procédé pour la préparation de composés de formule générale:

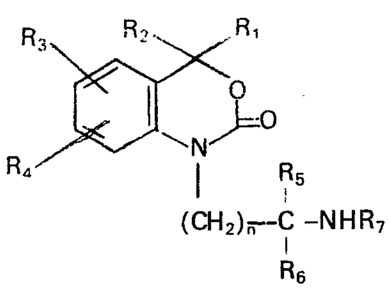

(I),

dans laquelle:

$R_1$ et $R_2$, qui peuvent être identiques ou différents, représentent hydrogène ou alcoyle en $C_1$–$C_4$,

$R_3$ et $R_4$, qui peuvent être identiques ou différents, représentent hydrogène, fluor, chlore, hydroxy, méthyle, éthyle, alcoxy en $C_1$–$C_4$ ou également ensemble méthylènedioxy,

$R_5$ et $R_6$, qui peuvent être identiques ou différents, représentent hydrogène ou méthyle,

$R_7$ représente le groupe

-CH(R)-CH(OH)— 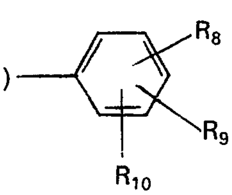 (II),

R représente hydrogène ou alcoyle en $C_1$–$C_4$,

$R_8$ représente fluor, chlore, alcoyle en $C_1$–$C_4$, alcoylthio en $C_1$–$C_4$, hydroxyméthyle ou l'un des groupes $CONHR_{11}$, $SO_2NHR_{11}$, $OR_{12}$, méthoxycarbonyle, éthoxycarbonyle ou $NHSO_2CH_3$,

$R_9$ représente hydrogène, fluor, chlore ou $OR_{12}$,

$R_{10}$ représente hydrogène, amino, méthyle, méthoxy ou chlore,

$R_{11}$ représente hydrogène, méthyle, éthyle ou hydroxyéthyle,

$R_{12}$ représente hydrogène, alcoyle en $C_1$–$C_4$, alcoyle en $C_1$–$C_4$–CO, $C_6H_5$–$CH_2$ ou $C_6H_5$–CO, et

n représente 1, 2 ou 3, sous forme de racémates, éventuellement de paires d'antipodes diastéréoisomères ainsi que d'énantiomères individuels, dans chaque cas sous forme de bases libres ou sous forme de sels d'addition d'acides, caractérisé en ce que:

a) on réduit un composé de formule générale:

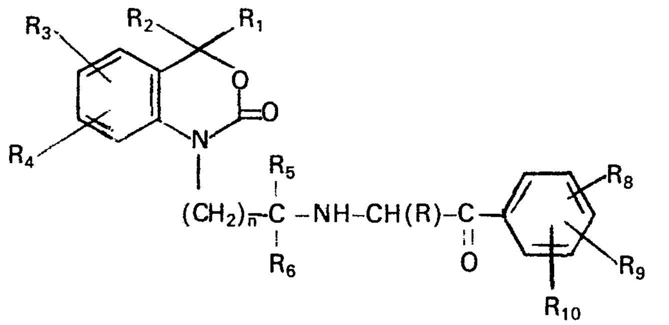

(II)

dans laquelle R, $R_1$–$R_{10}$ et n ont la signification ci-dessus, ou en ce que:

b) on alcoyle de façon réductrice un composé de formule générale:

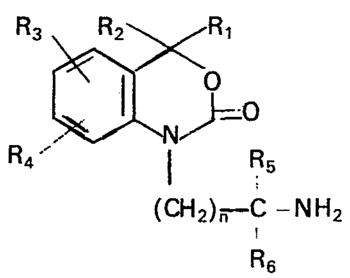

(III),

dans laquelle n et $R_2$–$R_6$ ont la signification ci-dessus, au moyen d'un composé de formule générale:

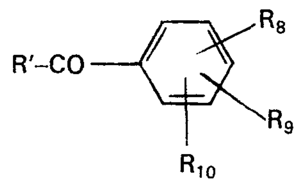

(IV),

dans laquelle $R_8$, $R_9$ et $R_{10}$ ont la signification ci-dessus, et R' remplace l'un des groupes:

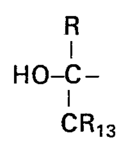

ou

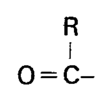

(Va) (Vb)

R aynat la signification ci-dessus et $R_{13}$ étant alcoyle en $C_1$–$C_4$, ou en ce que:

c) on clive le groupe protecteur $R_{14}$ à partir d'un composé de formule générale:

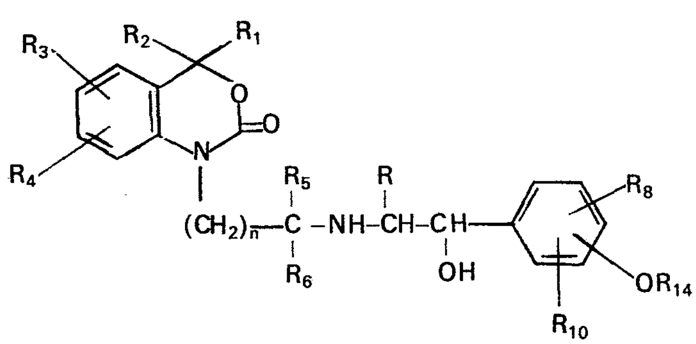

(VII)

dans laquelle n, R et $R_1$–$R_{10}$ ont la signification ci-dessus, et $R_{14}$ remplace benzyle,

CO-(alcoyle en $C_1$–$C_4$) ou CO–$C_6H_5$, ou en ce que:

d) on fait réagir un composé de formule générale:

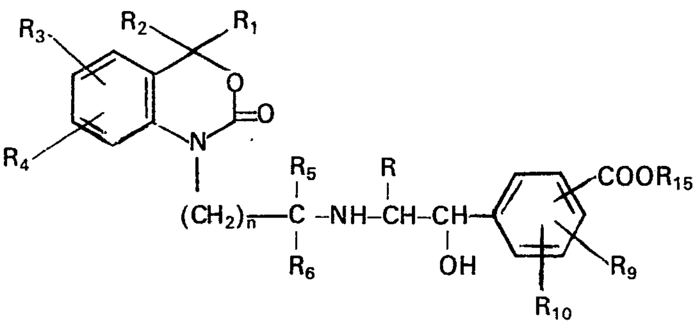

(IX),

dans laquelle n, R et $R_1$–$R_{10}$ ont la signification ci-dessus, et $R_{15}$ remplace un radical alcoyle en $C_1$–$C_4$ ou aralcoyle, avec une amine de formule générale:

$H_2NR_{11}$ (X)

dans laquelle $R_{11}$ a la signification ci-dessus, et en ce que, si on le désire, on sépare les produits obtenus selon les procédés a) à d) en paires d'antipodes diastéréoisomères ou en les énantiomères individuels, et/ou on transforme les bases obtenues en premier lieu en sels d'addition d'acides, les sels d'addition d'acides obtenus en premier lieu en bases libres.

**Claims**

1. Compounds of general formula

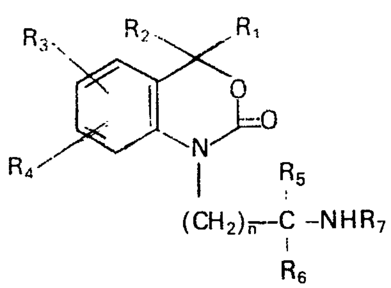

(I),

in which $R_1$ and $R_2$, which can be the same or different, represent hydrogen or ($C_1$–$C_4$) alkyl,

$R_3$ and $R_4$, which can be the same or different, represent hydrogen, fluorine, chlorine, hydroxy, methyl, ethyl, ($C_1$–$C_4$) alkoxy or together methylenedioxy,

$R_5$ and $R_6$, which can be the same or different, represent hydrogen or methyl,

$R_7$ represents the group

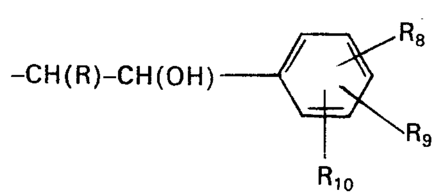

(II),

R represents hydrogen or ($C_1$–$C_4$) alkyl

$R_8$ represents fluorine, chlorine, ($C_1$–$C_4$) alkyl, ($C_1$–$C_4$) alkylthio, hydroxymethyl or one of the groups $CONHR_{11}$, $SO_2NHR_{11}$, $OR_{12}$, methoxycarbonyl, ethoxycarbonyl or $NHSO_2CH_3$, wherein

$R_9$ represents hydrogen, fluorine, chlorine or $OR_{12}$,

$R_{10}$ represents hydrogen, amino, methyl, methoxy or chlorine,

$R_{11}$ represents hydrogen, methyl, ethyl or hydroxyethyl,

$R_{12}$ represents hydrogen, ($C_1$–$C_4$) alkyl, ($C_1$–$C_4$) alkyl-CO, $C_6H_5$–$CH_2$ or $C_6H_5$–CO, and

n represents 1, 2 or 3, in the form of racemates, if appropriate diastereomeric antipodal pairs and individual enantiomers, each as free bases or as acid addition salts.

2. Compounds according to claim 1, characterised in that

$R_1/R_2$ represents $CH_3/CH_3$ or $CH_3/H$,

$R_3$ represents H, OH or $OCH_3$

$R_4$ represents H,

$R_5/R_6$ represents $CH_3/CH_3$, $CH_3/H$ or H/H,

$R_7$ represents $-CH(R)-CH(OH)-$ 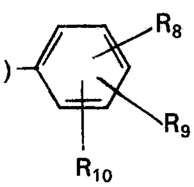

R represents H, $CH_3$ or $C_2H_5$

$R_8$ represents $CONHR_{11}$, $SO_2NHR_{11}$, $NHSO_2CH_3$, $OR_{12}$, F, Cl, COOAlkyl or $CH_2OH$,

$R_9$ represents $OR_{12}$, F, Cl

$R_{10}$ represents H, $NH_2$, $CH_3$, $OCH_3$ or Cl

$R_{11}$ represents H, $CH_3$ or $C_2H_5$

$R_{12}$ represents H, $C_6H_5$–$CH_2$ or $C_6H_5$–CO and

n represents 1 or 2.

3. Pharmaceutical, characterised by a content of a compound according to claim 1 or 2 in conjunction with conventional excipients and/or carriers.

4. Process for preparing compounds of general formula

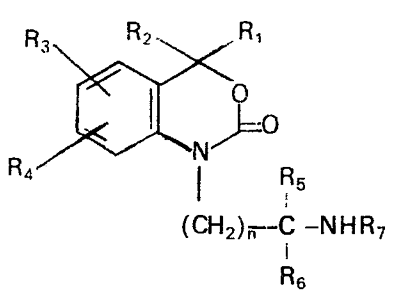

(I),

in which $R_1$ and $R_2$, which can be the same or different, represent hydrogen or $(C_1–C_4)$ alkyl,

$R_3$ and $R_4$ which can be the same or different represent hydrogen, fluorine, chlorine, hydroxy, methyl, ethyl, $(C_1–C_4)$ alkoxy or together methylendioxy,

$R_5$ and $R_6$ which can be the same or different represent hydrogen or methyl,

$R_7$ represents the group

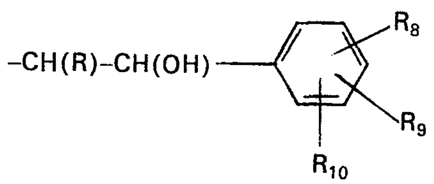

(II),

R represents hydrogen or $(C_1–C_4)$ alkyl,

$R_8$ represents fluorine, chlorine, $(C_1–C_4)$ alkyl, $(C_1–C_4)$ alkylthio, hydroxymethyl or one of the groups $CONHR_{11}$, $SO_2NHR_{11}$, $OR_{12}$, methoxycarbonyl, ethoxycarbonyl or $NHSO_2CH_3$,

$R_9$ represents hydrogen, fluorine, chlorine or $OR_{12}$,

$R_{10}$ represents hydrogen, amino, methyl, methoxy or chlorine,

$R_{11}$ represents hydrogen, methyl, ethyl or hydroxyethyl,

$R_{12}$ represents hydrogen, $(C_1–C_4)$ alkyl, $(C_1–C_4)$ alkyl-CO, $C_6H_5–CH_2$ or $C_6H_5–CO$, and

n represents 1, 2 or 3, in the form of racemates, if appropriate diastereomeric antipodal pairs and individual enantiomers, each as free bases or as acid addition salts, characterised in that

a) the compound of general formula

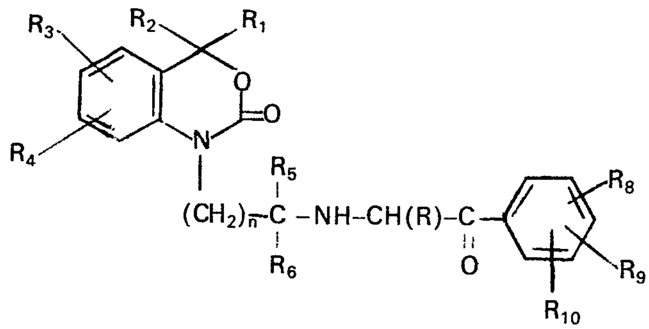

(II)

in which R, $R_1$–$R_{10}$ and n have the above meanings, is reduced, or in that

b) a compound of general formula

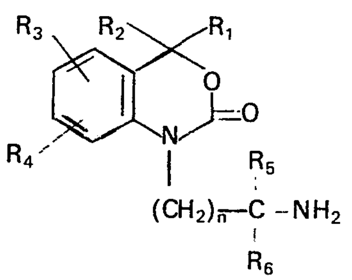

(III),

in which n and $R_2$–$R_6$ have the above meanings, is alkylated reductively with a compound of general formula

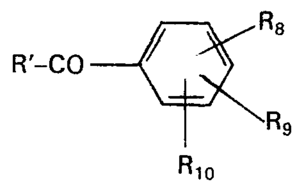

(IV),

in which $R_8$, $R_9$ and $R_{10}$ have the above meanings and R′ represents one of the groups

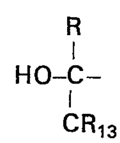

or

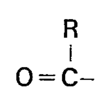

(Va) (Vb)

in which R has the above meaning and $R_{13}$ represents $C_1$–$C_4$ alkyl, or in that

c) The protective group $R_{14}$ is split off from a compound of general formula

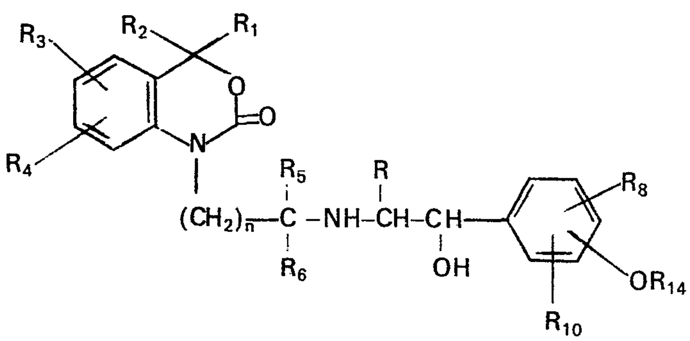

(VII)

in which n, R and $R_1$–$R_{10}$ have the above meanings and $R_{14}$ represents benzyl, CO–($C_1$–$C_4$ alkyl) or CO–$C_6H_5$, or in that

d) a compound of general formula

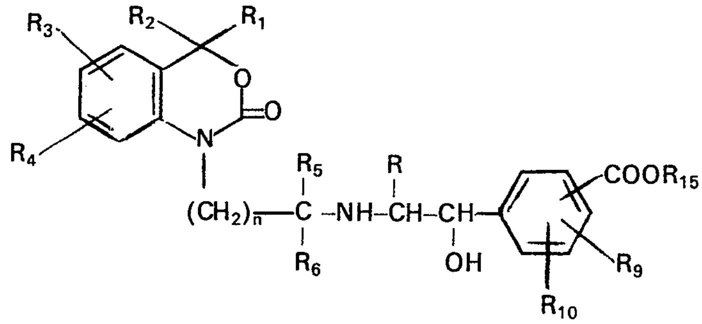

(IX),

in which n, R and $R_1$–$R_{10}$ have the above meanings and $R_{15}$ represents a ($C_1$–$C_4$) alkyl or aralkyl, is reacted with an amine of general formula

$H_2NR_{11}$ (X)

in which $R_{11}$ has the above meaning, and in that, if desired, products obtained according to processes a) to d) are separated into diastereomeric antipodal pairs or into the individual enantiomers and/or bases obtained primarily are converted into acid addition salts and acid addition salts obtained primarily are converted into free bases.